# EUROPEAN PATENT SPECIFICATION

(11) **EP 2 798 057 B1**
(45) Date of publication and mention of the grant of the patent: **22.08.2018**
(21) Application number: 12862569.6
(22) Date of filing: 28.12.2012
(51) Int. Cl.: C12N 5/073, A61L 27/36, A61K 35/35, A61K 35/36

(54) **REGENERATIVE TISSUE MATRIX**
REGENERATIVE GEWEBEMATRIX
MATRICE DE RÉGÉNÉRATION TISSULAIRE

(30) Priority: 30.12.2011 US 201161581803 P
(43) Date of publication of application: 05.11.2014
(73) Proprietor: VIVEX BIOMEDICAL, INC., Atlanta, GA 30339 (US)
(72) Inventor: MORRIS, Paul, R., Coral Gables, FL 33146 (US); KAST, Nathan, North Miami Beach, FL (US); TEMPLE, H., Thomas, Miami, FL 33156 (US); WOLFINBARGER, Lloyd, Norfolk, VA 23508 (US)
(74) Representative: Gislon, Gabriele
(86) International application number: PCT/US2012/072027
(87) International publication number: WO 2013/102048

(56) References cited:
- WO-A2-02/43778
- US-A1- 2003 087 428
- US-A1- 2004 005 703
- US-A1- 2005 013 870
- US-A1- 2005 025 838
- US-A1- 2008 299 171
- US-A1- 2008 306 610
- US-A1- 2011 045 044
- US-A1- 2011 045 045

## Description

### STATEMENT REGARDING FEDERALLY-SPONSORED RESEARCH AND DEVELOPMENT

Not applicable.

### BACKGROUND OF THE INVENTION

### Field of the Invention

This invention relates generally to the field of tissue matrices for regenerative repair. More particularly, this invention relates to methods for processing tissues for use in regenerative repair of soft tissue defects and the resulting tissue matrices as disclosed in claims 1 and 10.

### Description of Related Art

When damage to soft tissue occurs, an inflammatory response is initiated and cells and molecular factors are recruited to the site of injury to begin the healing process. In order for healing to occur, these cells must be able to infiltrate and proliferate into the wounded area.

Cellular proliferation in tissues is controlled by both positive and negative agents present within that tissue. Positive agents are present in tissue to stimulate cellular proliferation (mitogenic agents) and differentiation (morphogenic agents). Negative agents are present in tissue to stop cellular proliferation and/or cellular differentiation. It is this sensitive and balanced approach to regulating cellular activity in tissues that represents what, in certain instances, is a fine line between a non-neoplastic and a neoplastic, or cancerous, state. Regenerative medicine has become an important strategy involving the use of biological materials in the repair of tissue pathologies where the expectation of the regenerative aspects of the repair is to reduce the amounts of fibrous scar tissue formation which may close the pathology, but not further participate in the function of the tissue being regenerated. For instance, U.S. Patent No. 7,851,447 describes a method for nerve repair comprising one or more chondroitin sulfate proteoglycan (SCPG)-degrading enzymes (for example chondroitinase ABC) that promote axonal penetration into damaged nerve tissue.

WO02/43778 discloses a method for the preparation of a bioprosthetic tissue. The method includes removing phospholipid binding sites contained in a biological tissue, so that infectious agents or other unwanted substances are prevented or inhibited from binding to the tissue. The method comprises contacting a bioprosthetic tissue with a surfactant (e.g. Tween 80) as well as a denaturing agent, such as a protic solvent preparation (e.g. ethanol and isopropanol) having binding affinity for the infectious agent. Preferably, all phospholipids in a tissue are essentially removed. Since proteoglycans occur in infectious agent binding sites, the method removes binding sites having both a protein and a polysaccharide component, such as proteoglycan.

US 2003/0087428 discloses a process for producing acellular soft-tissue implants. The process includes extracting a soft-tissue sample with a solution comprising nonionic detergents to produce extracted tissue, treating the extracted tissue with a solution comprising anionic detergents, washing the treated tissue with a decontaminating solution to produce the acellular soft tissue graft; and storing the acellular soft tissue graft in a storage solution comprising decontaminating agents.

Further, U.S. Patent Nos, 7,008,763, 7,736,845, and 7,687,230 describe methods of treating collagenous connective tissues with the objective of that tissue being "re-habited" or "re-colonized" by host cells without immune rejection and inflammatory reaction. They describe a tissue soaked in cold temperatures with polyglycol, salt, hydrogen peroxide, and phosphate buffer with the objective of permitting ground substances to dissociate such that the collagen fibers remain stable. As a second processing solution, the tissues are soaked in an alcohol and water solution and/or solutions comprising anti-inflammatory and anti-thrombogenic agents.

As another example, many heart attacks are the result of occluded vasculature and the resultant ischemic damage to the cardiomyocytes comprising the contractile component of the heart. Efforts to repair this ischemic damage have been varied, such as efforts involving seeding of cells into the ischemically damaged areas with the objective of restoring functioning cardiomyocytes. For example, U.S. Patent No. 6,953,466 describes methods for the delivering of therapeutic implants to tissues where the therapeutic agents include vascular endothelial growth factor, fibroblast growth factor, platelet derived growth factor, and angiopoietins. In this particular method, the therapeutic agents are delivered via an elongate catheter and depositing the carrier and therapeutic agent in the tissue. A similar method is described in U.S. Patent No. 6,749,617; however, the method described in this patent includes the use of a solid or liquid carrier of the therapeutic agents and the therapeutic agents include cells. In this particular method, the carrier constitutes a biocompatible scaffold for the cells which will move from that scaffold into the tissue being repaired. A method for delivery of cellular materials into heart muscle is described in U.S. Patent No. 7,686,799 wherein the cellular materials are deployed directly into the heart muscle wall. U.S. Patent No. 7,892,829 specifically describes cardiac muscle regeneration using mesenchymal stem cells. These stem cells can be administered as a liquid injectable or as a preparation of cells in a matrix which is or becomes solid or semisolid.

These efforts to repair damaged heart muscle involve the use of liquid or solid carriers of therapeutic agents and although these therapeutic agents can include cells, these efforts do not depend on the use of a process to prepare the damaged heart muscle to receive cells via a natural ingrowth mechanism wherein the dead cellular remnants from the ischemic damage to the heart have been removed or modified in some manner to expose normally occurring molecular moieties present on a collagenous matrix or tissue matrix. The resultant outcome has mostly been the formation of scar tissue (a repair process) rather than the formation of functional cardiac muscle (a regeneration process). The currently available technologies fail to deal with the presence of agents in the damaged myocardium that will act in a negative manner towards the cells being added such that the cells become directed along a repair pathway leading to scar tissue formation rather than a regenerative pathway leading to restoration of functional cardiac muscle tissue.

There is a considerable body of literature and patents pertaining to a removal of cells from tissues to be used clinically and much of this material falls under a broad category of decellularization of tissues or rendering tissues acellular, and/or avital (lacking a vital cell population). Indeed, one of the earliest patents describing a process for the decellularization of tissues was by Brendel and Duhamel (U.S. Patent No. 4,801,299) which involved the use of nonionic detergents, hypotonic solutions, anionic detergents, DNAase and RNAase enzymes and protease inhibitors for the removal of cells from tissues. See also U.S. Patent Nos. 4,539,716; 4,546,500; 4,835,102; 4,776,853; 5,558,875; 5,843,181; 4,776,853; and 5,843,180 for examples of differing strategies for the decellularization of tissues. Most of these decellularization strategies focused on the production of decellularized tissues in which success was evidenced by the lack of visible cellular remnants in histological preparations. Indeed, U.S. Patent Nos. 5,613,982; 5,632,778; 5,843,182; and 5,899,936 claimed only a partial reduction in extractable nucleic acids in decellularized tissues with little evidence of other changes in lipid, phospholipid and/or proteoglycan compositions. Such tissues were described as having recellularized following implantation into a patient; however, initial outcomes indicated considerable calcification of the tissues without indicating the cause or corrective actions needed with this method for decellularizing tissues.

Similarly, U.S. Patent Nos. 6,743,574, 6,432,712, and 7,179,287 described decellularization methodologies involving the removal of cell remnants to a level that histologic evidence of cells was not visible in histology preparations. Pulmonary patch grafts processed according to U.S. Patent No. 6,743,574 have since received clearance based on a pre-market notification to the FDA, and such tissues have been shown to recellularize *in situ* with little indication of pathologic complications. Thus, specific processing of tissues to remove cellular elements from tissues have been tentatively identified as providing for tissues suitable for repair of soft tissue defects. However, to date such tissues have not been produced which will contribute to a regeneration of soft tissues to produce tissue materials that are functionally equivalent to the native tissue into which they are implanted.

A series of U.S. Patents (U.S. Patent Nos. 6,576,265; 6,893,666; 6,890,564; 6,890,563; 6,890,562; 6,887,495; 6,869,619; 6,861,074; 6,852,339; and 6,849,273) describe a process for producing a devitalized connective tissue for use in tissue repair and regeneration. However, the method used to produce this devitalized tissue is only generally described as involving the use of hypotonic saline and peroxides. Moreover, it is not sufficient to merely decellularize a tissue, nor is sufficient to simply add agents to a processed tissue that act in a positive manner to stimulate cellular proliferation and differentiation.

Thus, the field of regenerative medicine is still in need of a tissue that will be effective in regeneration, rather than repair, of damaged soft tissues, and methods of creating the same. It is not sufficient to just decellularize a tissue, nor is sufficient to just add agents to a processed tissue that act in a positive manner to stimulate cellular proliferation and differentiation.

### SUMMARY OF THE INVENTION

The present invention is directed to a process of preparing a tissue matrix which is suitable for regenerative repair of soft tissue, as well as being directed to such a tissue matrix and kits therefor as described in the claims. The present invention is directed to a process for the preparation of a tissue matrix suitable for regenerative repair of tissues which includes steps of isolating a portion of connective tissue for use as the tissue matrix, and contacting the connective tissue with a surfactant and with a disinfectant. This results in reducing the levels of at least one of proteoglycans, lipids, phospholipids, nucleic acids, major histocompatibility (MHC) antigens (e.g., MHC I or MHC II), contaminating microorganisms, and measured endotoxins. While many of these elements are significantly reduced, such as generally up to 90% or 99%, a substantial amount of extractable proteoglycans are generally retained, such as 70% of extractable proteoglycans. The process results in the production of a tissue matrix that facilitates recellularization and regenerative repair in *in vitro* and *in vivo* applications.

In some embodiments, the process also includes contacting the connective tissue with a chondroitinase, alcohol, endonuclease, and/or lipase to further reduce certain inhibitory agents from the connective tissue matrix. Moreover, the process may include dehydrating and/or freeze-drying the resultant tissue matrix for storage and/or transport.

In at least one embodiment, the process of the present invention includes contacting an isolated portion of connective tissue with one or more solutions capable of reducing phospholipids, lipids, proteoglycans, nucleic acids, major histocompatibility antigens (MHC) I and II, contaminating microorganisms, and endotoxins. The resultant tissue matrix retains molecular signals appropriate to recellularization in *in vitro* and *in vivo* applications.

The present invention is also directed to a tissue matrix as produced by the instant method. The tissue matrix of the present invention is suitable for use in regenerative repair of soft tissue. Such tissue matrix includes a scaffold portion structured to provide shape to the tissue matrix, and may be made of structural proteoglycans and have a collagenous structure. The tissue matrix also includes a non-structural portion disposed in interspersed relation through at least a portion of the scaffold or structural portion, and which may include non-structural proteoglycans. The various portions of the tissue matrix are collectively structured to promote cellular infiltration, attachment, and proliferation of host cells into the matrix upon tissue implantation or transplantation into a host. For example, the tissue matrix is reduced in levels of phospholipids, lipids, nucleic acids, major histocompatibility antigens (MHC) I and II, contaminating microorganisms, and endotoxins, and to some degree proteoglycans, and in some embodiments are at least partially acellularized.

The present invention is also directed to kits for regenerative soft tissue repair having at least a tissue matrix as described herein for implantation or use, as well as kits for processing connective tissue to render a resultant tissue matrix suitable for regenerative soft tissue repair, including at least an amount of surfactant and an amount of disinfectant.

The processes, tissue matrices, and kits herein described can be used in connection with pharmaceutical, medical, and veterinary applications, as well as fundamental scientific research and methodologies, as would be identifiable by a skilled person upon reading of the present disclosure.

### BRIEF DESCRIPTION OF THE DRAWINGS

For a fuller understanding of the nature of the present invention, reference should be had to the following detailed description taken in connection with the accompanying figures in which:
Figure 1 shows attachment and proliferation of L-929 cells on dermal matrix processed from donor UPS9571. When plated at a low density (10000 cells/sample), L-929 cells were barely detectable at early time point (data not shown), but formed a nice layer of cells on both sides of the samples after 36 days (A, B, C, D). B and D: basement membrane side. Scale bars: 100 µm.
Figure 2 shows attachment of L-929 cells on dermal matrix processed from donor UPS9238. When plated at a higher density (200000 cells/sample), L-929 cells were easily observable as early as 2 days after plating (A, B) and formed a thick layer of cells at day 5 (C, D). At day 22, infiltration of cells in the tissue was observable (E, F, G, H). Scale bars: 100 µm.
Figure 3 shows attachment of proprietary MIAMI cells on dermal matrix dermis processed from donor UIN 9611. When plated at a low density (10000 cells/sample), MIAMI cells were observable 35 days after plating (A black arrows, B, C) both on the surface and inside the dermal matrix. Scale bars: 100 µm.
Figure 4 shows DNA extracted from dermal matrix and quantified, comparing dermal matrix as treated with the process of the present invention and a non-treated control. Significantly less DNA was extracted from treated dermal matrix, indicating the process of the instant invention is effective at reducing nucleic acids in the matrix.
Figure 5 shows a calibration curve of the fluorescence signal related to the number of viable cells.
Figure 6 shows the toxicity assessment of ilio-tibial band tissue.
Figure 7 shows L-929 cell morphology at the end of a cellular inhibition assay. Note that control cells plated at 20k cells per cubic centimeter at day 1 were too confluent at day 3 (E). Scale bars: 100 µm.
Figure 8 shows the biocompatibility of micronized dermal matrix seeded with L-929 cells, indicating cell proliferation over time.
Figure 9 shows a calibration curve of the fluorescence signal related to the number of viable L-929 cells seeded on micronized dermal matrix, indicating that these cells remain viable.
Figure 10 shows L-929 cell morphology during the biocompatibility assay at Day 10. Cells appeared healthy and aggregated on the surface of the micronized dermis (A-C). Detail at 200X (B). Negative control: UMTB micronized dermis 25-300 µm (D) .
Figure 11 shows L-929 cell morphology during the biocompatibility assay at Day 16. Cells appeared healthy and aggregated on the surface of the micronized dermis (A-C). Negative control: UMTB micronized dermis 25-300 µm (D).
Figure 12 shows L-929 cell morphology during the biocompatibility assay at Day 30. Cells appeared healthy and aggregated on the surface of the micronized dermis (A-C). Negative control: UMTB micronized dermis 25-300 µm (D).
Figure 13 shows wound healing on a first patient using a dermal matrix graft. At Day 0 (A), a 4x4cm dermal matrix graft was placed on the wound. At Day 12 (B), development of granulation tissue is seen below the antibiotic ointment, as indicated by the arrow. By Day 40 (C), the wounded tissue has almost completely regenerated.
Figure 14 shows wound healing on a second patient using a dermal matrix graft. Panel A shows a post-radiation wound dehiscence graft at Day 1. Panel B shows Day 2 post graft placement. Panel C shows Day 30 post graft placement, wherein the wounded tissue is almost completely regenerated.

### DETAILED DESCRIPTION

The present invention is directed to processes for treating connective tissue to prepare a tissue matrix suitable for use in regenerative soft tissue repair, as well as such tissue matrix and kits therefor.

Unless otherwise defined, all terms of art, notations and other scientific terms or terminology used herein are intended to have the meanings commonly understood by those of skill in the art to which this invention pertains. In some cases, terms with commonly understood meanings are defined herein for clarity and/or for ready reference, and the inclusion of such definitions herein should not necessarily be construed to represent a substantial difference over what is generally understood in the art. It will be further understood that terms, such as those defined in commonly used dictionaries, should be interpreted as having a meaning that is consistent with their meaning in the context of the relevant art and/or as otherwise defined herein.

The terminology used herein is for the purpose of describing particular embodiments only and is not intended to be limiting of the invention. As used herein, the indefinite articles "a", "an" and "the" should be understood to include plural reference unless the context clearly indicates otherwise.

The phrase "and/or," as used herein, should be understood to mean "either or both" of the elements so conjoined, i.e., elements that are conjunctively present in some cases and disjunctively present in other cases.

As used herein, "or" should be understood to have the same meaning as "and/or" as defined above. For example, when separating a listing of items, "and/or" or "or" shall be interpreted as being inclusive, i.e., the inclusion of at least one, but also including more than one, of a number of items, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e., "one or the other but not both") when preceded by terms of exclusivity, such as "either," "one of," "only one of," or "exactly one of."

As used herein, the terms "including", "includes", "having", "has", "with", or variants thereof, are intended to be inclusive similar to the term "comprising."

As would be understood by those of skill in the art, the processes, tissue matrices, and kits of the present invention are applicable to the regenerative repair of tissues.

The present invention is directed to a process for preparing a tissue matrix suitable for regenerative repair of soft tissue. In at least one embodiment, the process includes isolating a portion of connective tissue for use as the tissue matrix. Any appropriate connective tissue may be used, such as but not limited to dermis, fascia, skin, pericardium, periosteum, tendon, ligament, dura, omentum, cartilage, and combinations thereof. The connective tissue may be allogenic, autogenic or xenogenic, and may be isolated from a cadaver or living donor. Connective tissues to be processed by the methods herein may be first procured or harvested from a human or animal donor and immediately placed in a stabilizing transportation solution which arrests and restricts autolytic and proteolytic degradation, protects against bacterial and fungal contamination, and reduces mechanical damage. The stabilizing solution generally contains an appropriate buffer, one or more antibiotics, and is transported at the temperatures of wet ice. Non-limiting examples of components of a stabilizing solution include a cold solution (generally about 1 to about 10 degrees Celsius) of lactated ringer, sodium bicarbonate and antibiotics (e.g., gentamicin, vancomycin, etc.).

Once isolated, the process includes the steps of contacting the connective tissue with at least one solution capable of significantly reducing molecular elements which are inhibitory to cellular ingrowth and proliferation, which includes at least one component selected from the group consisting of lipids, phospholipids, nucleic acids, major histocompatibility antigens, endotoxins, and contaminating microorganisms. It will also at least partially reduce the proteoglycan content of the connective tissue. In at least one embodiment, the process includes contacting the connective tissue with a surfactant, such as a detergent, as well as contacting the connective tissue with a disinfectant, described in greater detail hereinafter.

All solutions utilized in the process of the invention will reduce "proteoglycan content". "Proteoglycan content" as used herein refers to structural proteoglycans, soft filler proteoglycans, chondroitins, hyaluronans, proteins, polysaccharides, etc. All processing steps will more readily extract these components residing in the non-structural aspects of the tissue, but steps including disinfectants, such as peracetic acid, will work equally well on proteoglycans in both the structural and non-structural (i.e. soft filler) aspects of the tissue. In other embodiments, however, the process includes contacting a tissue with at least one chondroitinase enzyme, such as but not limited to chondroitinase ABC, to significantly reducing the presence of particular proteoglycans, such as chondroitin sulfate.

Proteoglycans contain covalently linked "chondroitins." Proteoglycans forming the structural aspect of the tissue are generally not easily extracted by the present invention, but can be chemically modified by the present invention. For proteoglycans not forming the structural aspect of the tissue, the present invention will not only serve to extract, but to chemically modify, by oxidizing, these proteoglycans as well as the non-structural chondroitins, hyluronins, and other smaller molecular weight proteins and polysaccharides and high molecular weight nucleic acids.

Extractable proteoglycans are primarily chondroitins and hyaluronans associated with proteins not yet incorporated into the structural aspects of the tissue (i.e. residing within the "soft filler" aspect of the tissue). Care should be taken not to remove too much proteoglycan content. Allowable ranges of removal of extractable "proteoglycans" (i.e., chondroitins, hyaluronans, proteins, etc.) wherein maximum reductions in extractable "proteoglycans" should not be greater than 50%, nor less than 30%. Sufficient extraction of the extractable "proteoglycans" are necessary to reduce the "soft filler" contained within the structural elements of the tissue such that cells can infiltrate, but not so much as to render the tissue matrix unsuitable to cells entering the tissue matrix. Illustrative of this point, tissue extracellular matrix is normally very viscous and a nominal reduction in this viscosity will aid in cellular movement into the now less viscous environment but still retain a sufficient physical nature that cells will have a solution adjacent to their plasma membranes that will prevent molecules the cells are producing and secreting from diffusing rapidly away from them.

As such, the process may result in retention of a substantial amount of extractable proteoglycans, which help promote cell-cell and cell-matrix interactions. The process results in production of a tissue matrix that facilitates recellularization and regenerative repair in *in vitro* and *in vivo* applications. Retention of proteoglycans, matrix proteins such as collagen, laminin and elastin, as well as the glycoprotein vitronectin are all important in cell binding and promote tissue remodeling. The process of preparation of the tissue matrix does reduce many components of the extracellular matrix (ECM) including glycosaminoglycans (GAGs), which are covalently linked to protein to form proteoglycans. GAGs such as hyaluronan, keratin sulfate, and heparin sulfate are an integral component of the ECM and play an important role in cell-cell and cell-matrix interactions, and assist with cell migration and differentiation. Thus, in a preferred embodiment, the tissue matrix retains about 70% of extractable proteoglycans.

As described throughout the specification, the process of the present invention includes steps of contacting the connective tissue with various solutions. As used herein, "contacting" may include application of the solution to only a portion of the connective tissue, but also may include immersion of the connective tissue in the solution, such that the tissue is completely covered thereby. In at least one embodiment, immersion may be static, in which the connective tissue is covered by the solution and is not moved. In other embodiments, immersion is dynamic, which includes covering with a solution as well as includes stirring, rocking, agitation, ultrasonic energy, or other energy or movement being imparted on the connective tissue while it is immersed. Furthermore, the process may include rinsing the connective tissue with one or more rinsing or cleansing solutions, such as saline, buffered saline, or water, to rinse the tissue at various steps in the process to remove any previously used solutions.

The solutions utilized in the process of the present invention include at least a surfactant, such as a detergent, and a disinfectant. Surfactants will break down lipid and phospholipid content, and have also have molecular impacts on endotoxins and MHC antigens to help reduce those as well. Surfactants and detergents may be anionic, nonionic or cationic. Examples of useful surfactants and/or detergents include, but are not limited to, BRIJ 95 detergent, BRIJ 96 detergent, BRIJ 98 detergent, Triton X-100, Tween 20, and combinations thereof.

Disinfectants, and optionally other biocidal agents, are used in the instant process for more than just their microbe killing abilities. Disinfectants are very damaging agents, and will significantly reduce levels of lipids, phospholipids, nucleic acids, MHC antigens, endotoxins, and contaminating microorganisms from the connective tissue being treated. Examples of disinfectants useful in the present process include, but are not limited to: peracetic acid, chlorine dioxide, hydrogen peroxide, polyvinylpyrolidineiodide, formaldehyde, glutaraldehyde, phenoxyethanol, methylparaben, propylparaben, sodium hydroxymethylglycinate, diazolidinyl urea, DMDM hydantoin, iodopropynyl butylcarbamate, propylene glycol, and combinations thereof. In at least one preferred embodiment, peracetic acid is used as a disinfectant. Peracetic acid is a potent antibacterial, antiviral, and antifungal disinfectant.

The disinfectant processing solution, such as peracetic acid, is the most effective solution at reducing nucleic acid content. In the case of peracetic acid, this may be due, at least in part, to the oxidative capacity of the peracetic acid in degrading the size of the nucleic acid polymers rendering them more soluble in aqueous solution and more likely to diffuse from the tissue during processing. Of the optional processing solutions, the endotoxin reducing solutions are the most aggressive for reducing nucleic acid content, and they work by degrading the nucleic acids to their small molecular weight components which will readily diffuse from the tissue during all subsequent processing steps. In some embodiments, the process also includes contacting the connective tissue an endonuclease. An endonuclease (s) may also optionally be used in some embodiments following either the detergent or peracetic acid treatment steps. For example, Benzonase®, OmniCleave™, Pulmozyme® (dornase alfa), and other broad spectrum endonucleases are useful in the present invention to target nucleic acids for destruction.

The disinfectant step of the processes described herein is also the most effective at reducing histocompatibility antigens. For instance, in the case of peracetic acid, the oxidative capacity of the peracetic acid solution accomplishes this result. However, chondroitinases such as chondroitinase ABC are also very effective, by enzymatically degrading the very cell surface polysaccharides of which the MHC I and II antigens are comprised.

Reduction of nucleic acids and major histocompatibility antigens in the steps of the processes of the present invention is useful in the significant reduction of immune responses, and possible tissue rejection, in a host receiving the processed tissue matrix. Nucleic acids that may be reduced include deoxyribonucleic acids, ribonucleic acids, and combinations thereof. Derivatives of both deoxyribonucleic acids and ribonucleic acids may additionally be reduced by the processes involved.

The disinfectant additionally contributes to the disruption of molecular structures and moieties, such as collagen, and also acts as a powerful oxidant of structural elements of the extracellular matrix (ECM) of the connective tissue being treated. Using peracetic acid as an example, the acetic acid component of peracetic acid alters the charge distribution of sulfated polysaccharides, resulting in a disruption of the molecular structures of the non-structural elements (i.e., "soft filler") (e.g., chondroitin sulfates, dermatin sulfates, hyaluronins, tropocollagens, etc.) and structural elements (e.g., fibrous collagens, elastins, proteoglycans, etc.) of the extracellular matrix of tissues. The oxidative properties of peracetic acid result in oxidation of groups (e.g., alcohol groups to aldehyde groups, amine groups to nitrate groups, etc.) and breakage of covalent bonds. Collectively, such properties of peracetic acid help solubilize the non-structural elements and also alter/solubilize the structural characteristics of the structural elements of tissues.

More specifically, the oxidation of molecular moieties by the peracetic acid steps herein (1) alters the antigenic nature of the major histocompatibility antigens by chemically modifying the sugars of the polysaccharide part of the MHC I and II antigens, (2) alters the ionic charge distribution on chondroitins and hyaluronins facilitating their solubilization, (3) alters the structural interactions between the structural proteoglycans (mostly contributing to intra- and intermolecular cross links) rendering the tissues less resorbable post implantation and strengthening them to tensile stress and strain (making them more manageable for handling and clinical application to a wound site). The oxidative reactions also serve to (4) degrade lipids and phospholipids by breaking the bonds at the alcohol/phosphate covalent linkage of glycerol to a phosphotidylacyl (or serine, etc.) group. The oxidative reaction will also (5) break the O-glycosidic linkages which link polysaccharides (chondroitins, hyaluronins, etc.) to proteins (i.e. breaks down proteoglycans). One aspect of this proteoglycan breakdown has to do with reduction of the antigenic components (the polysaccharide part of the MHC I and II antigens in cell membranes) of the major histocompatibility antigens, thus preventing an inflammatory response.

Therefore, one can reduce the viscosity of the soft tissue elements, by improving solubility and by degradation to smaller molecules, through both the disruption of the non-structural elements (i.e., chondroitin sulfates, dermatin sulfates, etc.) and by chemically altering the charge distribution of those molecules not extracted from the tissue. The objective is to reduce this soft tissue element viscosity and to change the molecular structure/nature of non-structural elements, which will change the molecular moieties that interact with cell surface receptors of cells trying to attach, proliferate, and infiltrate into the tissue, whether implanted into the body or in *in vitro* cell culture conditions.

The degree or extent of oxidation of molecular moieties within the tissue matrix may be quantified as a percentage (or number of oxidizable groups per unit wet/dry weight of the tissue) of the original oxidizable molecular moieties using low concentrations of a peroxide wherein the reduction of peroxide concentration(s) may be accurately determined using standard assays for peroxides. The mechanical properties (for example suture pull out strength) of the tissue matrix may be determined using standard mechanical tensile strength testing methodologies.

Non-limiting examples of some embodiments of concentrations and contacting times are provided below. Peracetic acid may be used for contacting in the processes provided as 0.05%, 0.1%, 0.5% solutions for 8 hours, 4 hours, and 2 hours, respectively. BRIJ 95 may be used for contacting in the processes provided as 0.25%, 0.5%, and 1.0% solutions for 48 hours, 24 hours, and 12 hours, respectively. Triton X-100 could alternatively be used instead of BRIJ 95 as a 0.25%, 0.5%, or 1.0% solution for contacting for 48 hours, 24 hours and 12 hours, respectively. Alcohols may be used for contacting as about 50% to about 85% solutions for about 1-2 hours. Furthermore, when NaCl is utilized, it may be used in contacting solutions at 1M for 48 hours, 1.5M for 24 hours, or 2M for 18 hours.

Tissues may also be aseptically processed through solutions of one or more type of antibiotic, antiviral, antifungal, or combinations thereof to reduce contaminating microorganisms. Examples include detergent, peracetic acid, and/or alcohol. This will render the tissue negative with respect to contaminating microorganisms. The desired times and conditions using the desired concentrations of processing solutions for aseptic treatment are described in more detail hereinafter. Processing solutions may optionally be removed between processing steps using saline, such as phosphate buffered saline (PBS), 1.5M NaCl, and 0.9% NaCl, or water rinsing solutions.

In some embodiments, the process also includes contacting the connective tissue a chondroitinase. Chondroitinase enzymes may optionally be used in some embodiments following the detergent treatment steps, to degrade one or more chondroitin sulfate proteoglycans, which are purported to inhibit axonal growth through a processed allograft nerve being used in the repair of severed or damaged nerves of the peripheral nervous system. One example of a chondroitinase enzyme is chondroitinase ABC, although other chondroitinases are contemplated.

The process may also include contacting the tissue with an endotoxin reducing solution, rendering the resultant tissue negative with respect to tests for endotoxins. In some embodiments, there are two processes that contribute to render the modified matrix negative for detectable endotoxins. The first is the oxidation and degradation of the lipopolysaccharides (LPS) endotoxin. Dilution of the endotoxin through multiple rinsing steps accounts for its further reduction. Such rinsing steps may include one or more of the following: a 0.1% peracetic acid solution, a 1.5M NaCl solution, a 0.5% BRIJ 35 solution, and combinations thereof. Disinfectants such as peracetic acid, as well as lipases, and chondroitinases will also serve to reduce the endotoxin levels mostly by removing the moieties on the endotoxins recognized by assay methods known in the art and in so doing will also reduce the potential for any remaining endotoxins from eliciting an inflammatory response when tissues are implanted.

In some embodiments, the process also includes contacting the connective tissue an alcohol. For instance, alcohols are useful further assisting in the breaking down of proteoglycans, lipids, phospholipids, nucleic acids, MHC antigens, endotoxins, and contaminating microorganisms. Examples of alcohols that may be used include, but are not limited to ethanol, propanol, isopropanol, butanol, glycerol, methanol, pentanol, and combinations thereof. The alcohol treatment step provides for a modest solubilization and extraction of phospholipids and lipids from tissues. It is well established in the art that 70% ethanol/isopropanol is maximally effective in solubilizing lipids. Hence, it is valuable as a disinfectant for Gram negative bacteria, where the lipids present in the bacterial cell walls make such Gram negative microorganisms more resistant to certain antibiotics and biocidal agents. Similarly, lipase enzymes will degrade lipids and chondroitinase (e.g., chondroitinase ABC) will degrade chondroitins, to which lipids and phospholipids may be intimately associated.

Reducible lipids of the processes described herein include nonsaponifiable lipids, or derivatives thereof, including cholesterols, derivatives of cholesterols, and combinations thereof. Reducible phospholipids or derivatives thereof include diacylglycerophosphatides, triacylglycerides and combinations thereof. Lipids and phospholipids tend to form micellar structures in aqueous solutions and thus tend to cover molecular moieties which infiltrating cells will need to "recognize" (via their cell surface receptors); and thus, it is important to remove as much of the lipid/phospholipid content as possible. In some embodiments, the process therefore also includes contacting the connective tissue a lipase, to augment lipid reduction. This may be desired when treating high lipid and phospholipid content connective tissues, such as dermal tissues, and particularly in processing thick dermal tissues.

Once treated, the process includes dehydrating the resultant tissue matrix for storage and/or transport. For example, the tissue matrix may be freeze-dried, such as by lyophilization or other cryogenic method, to reduce the residual moisture content of the tissue matrix to between about 2% and about 6%. Since such tissues may be more brittle than desired, in some embodiments the tissues may first be treated with a 10% to 30% solution of glycerol for a sufficient period of time to allow the preservation or storage solution to replace the water content of the processed tissues, and then freeze-dried to a residual moisture content of between 2% and 6%. Such tissues will be less brittle than similar tissues not being pretreated with glycerol prior to freeze-drying, and such tissues are also better preserved and better protected from any subsequent exposure to gamma irradiation used in some terminal sterilization steps. The tissue matrix may then be stored in a storage solution, which is preferably non-aqueous, such as mineral oil or glycerol.

In some embodiments, the processed tissue may be broken into smaller pieces from the hydrated, the freeze-dried, the preserved hydrated, or the preserved dehydrated state. This breaking into small pieces may be accomplished by standard methods and established means known to those skilled in the art, such as using shear forces in a mechanical blender, impact fragmentation, cryo-fracturing, and/or simple mechanical fragmentation using a mortar and pestle. In at least one embodiment, the tissue matrix is micronized, resulting in a plurality of fragments or pieces measuring in the micron range. For instance, micronized tissue pieces may have a diameter in the range of 100 - 300 microns, although some fragments may be less than 100 microns in average diameter. This micronized or fragmented tissue matrix may also be provided in a frozen state, a room temperature dehydrated state, a freeze-dried state, or a room temperature hydrated state. This micronized tissue matrix may later be used as an injectable tissue matrix, for subcutaneous regenerative repair, such as for use in cosmetic applications as just one illustrative example.

These above steps result in the modification of the tissue matrix sufficient to promote cellular infiltration and proliferation. For example, the present process involves reducing levels of at least proteoglycans, lipids, phospholipids, nucleic acids, and MHC antigens. As discussed elsewhere in this specification, proteoglycan levels are reduced by a range of 30% to 50%, such that the tissue matrix retains 50% to 70% of extractable proteoglycans. Lipids and phospholipids are reduced by a range of 70% to 95%. Nucleic acids are reduced by a range of 30% to 90%. Major histocompatibility (MHC) antigens I and II are reduced by a range of 85% to 99%. Moreover, endotoxins are essentially eliminated from the tissue matrix.

These results may be assessed by comparing the ability of standard mammalian cells to infiltrate non-processed connective tissues relative to connective tissues processed according to the present invention. Cellular infiltration can be assessed using traditional histological evaluation and/or by extracting nucleic acids from non-processed and processed tissues as a function of time of incubation in the presence of a test population of mammalian cells cultured under standard tissue culture conditions. An increase in extractable nucleic acids over time is indicative of cellular infiltration and proliferation. The Examples given hereinafter demonstrate confirmation by some of these tests.

The present invention is also directed to a tissue matrix suitable for use in regenerative repair of soft tissue, which may be a result of the previously described process in some embodiments. Specifically, the tissue matrix of the present invention comprises a scaffold portion structured to provide shape to the tissue matrix. For example, the scaffold portion may be made of structural or non-extractable proteoglycans. In some embodiments, the matrix is made up of a collagenous structure derived from connective tissues. The collagenous structure is suitable for cellular infiltration, cellular attachment, cellular proliferation, and cellular differentiation in both *in vivo* and *in vitro* conditions. The collagenous structure of the tissue matrix may possess hemostatic properties when used in both *in vivo* and *in vitro* conditions.

The tissue matrix also includes a non-structural portion disposed in interspersed relation through at least a part of the scaffold portion. For instance, the non-structural portion of the tissue matrix is the extracellular matrix (ECM) component of the tissue. As previously described, it permeates the scaffold and includes cells and molecular factors necessary for cell-cell and cell-matrix interaction that facilitate cellular infiltration, attachment, and proliferation. The non-structural portion of the tissue matrix may also be made up of proteoglycans, such as extractable and non-extractable proteoglycans.

The scaffold portion and non-structural portion of the tissue matrix are collectively structured to promote cellular infiltration, attachment, and proliferation of at least one host cell into the tissue matrix upon implantation or transplantation of the tissue matrix into a host tissue for repair.

Furthermore, the tissue matrix may retain molecular moieties associated with molecules comprising the collagenous structure appropriate to the attachment of mammalian cells. Particularly, the tissue matrix is composed of connective tissue with significant reduction in at least one of lipids, phospholipids, nucleic acids, major histocompatibility antigens, endotoxis, and some proteoglycans. Accordingly, at least part of the tissue matrix, either in the scaffold portion or the non-structural portion, have been at least partially acellularized by the process of removing these components. The tissue matrix nevertheless retains a significant amount of extractable proteoglycans. Preferably, the tissue matrix retains about 70% of extractable proteoglycans.

In some embodiments, the tissue matrix may also retain endogenously derived growth and differentiation factors. Such growth and differentiation factors include, but are not limited to, angiogenic factors, mitogenic factors, morphogenic factors, and combinations thereof. Angiogenic factors include, but are not limited to, one or more factors appropriate for the revascularization of a tissue matrix under *in vivo* and *in vitro* conditions. Likewise, mitogenic factors include, but are not limited to, one or more factors appropriate for induction of cells to divide mitotically, thereby proliferating and increasing in numbers. Morphogenic factors include, but are not limited to, one or more factors appropriate for the induction of cells infiltrating the tissue matrix to differentiate into cell phenotypes suitable to the formation of tissues of similar structure and function as the tissues into which the tissue matrix is placed.

Accordingly, the tissue matrix of the present invention meets one or more of the following criteria: (1) it is reduced by at least about 30%, and preferably greater than about 90%, in extractable nucleic acids; (2) it is reduced by about 70% to about 95% in extractable phospholipids; (3) it is reduced by about 70% to about 95% in extractable lipids; (4) it retains about 50% to about 70% of extractable proteoglycans; (5) it presents molecular moieties which have been oxidized; (6) it is reduced by at least about 85% in immunoreactive major histocompatibility antigens; (7) it presents a slightly altered collagenous structure which has been partially dissociated by the organic acid (preferably acetic acid); (8) it is disinfected and is culture negative according to USP Section 71 sterility assessments; (9) it is negative (i.e., not detectable by assay methods utilized by those skilled in the art or within the limits currently allowed by regulatory agencies) with respect to clinically acceptable levels of endotoxins; (10) it retains molecular moieties associated with the collagenous tissue matrix as appropriate to the needs of cells infiltrating the tissue matrix, and (11) it facilitates cellular infiltration, attachment, proliferation, differentiation, and synthesis of matrix materials appropriate to the regenerative repair of soft tissue defects.

The present invention is also directed to kits for regenerative soft tissue repair, which include a tissue matrix of isolated connective tissue as previously described. For instance, such kits include a tissue matrix having reduced levels of at least one of proteoglycans, lipids, phospholipids, nucleic acids, major histocompatibility (MHC) antigens, and endotoxins. The tissue matrix provided by the kit is therefore structured to promote cellular infiltration, attachment and proliferation of host cells therein. Moreover, the kits of the present invention may include tissue matrices of any type of tissue as described herein, such as but not limited to dermis and fascia. The tissue matrix may comprise any appropriate structure and dimension suitable to a particular repair purpose, such as but not limited to a sheet, thick sheet (as is readily understood in the art by those of ordinary skill), fragmented and/or micronized matrix, and which are ready to use in implantation.

In at least one embodiment, the kit further includes an amount of transfer solution, which is to be used in facilitating or assisting in the transfer of the tissue matrix to a host. Such transfer solution may be any appropriate biocompatible solution, such as but not limited to saline, buffered saline, or water. In addition, various instrumentation may be included in some embodiments of the kit to accomplish the transfer or implantation of the tissue matrix to a host. For example, the kit may include a needle and at least one syringe in which the tissue matrix may be loaded for subcutaneous injection into the host. This is particularly useful in connection with micronized tissue matrix, wherein the micronized tissue matrix may be suspended in transfer solution provided in the kit and injected into the host. A plurality of syringes and/or needles may be included in the kit for repeated injections. In some embodiments, the kit is available with the syringe pre-loaded with tissue matrix and transfer solution. In other embodiments, the tissue matrix, transfer solution, and syringe are separate in the kit and must be combined by a user.

The present invention is also directed to kits for processing connective tissue to render a resultant tissue matrix suitable for regenerative repair. Such kits include an amount of surfactant and an amount of disinfectant. As discussed previously, the surfactant may be a detergent, such as BRIJ 95, BRIJ 96, Tween 20, Triton X100 or others. The disinfectant may be peracetic acid peracetic acid, chlorine dioxide, hydrogen peroxide, polyvinylpyrolidineiodide, formaldehyde, glutaraldehyde, phenoxyethanol, methylparaben, propylparaben, sodium hydroxymethylglycinate, diazolidinyl urea, DMDM hydantoin, iodopropynyl butylcarbamate, propylene glycol, and combinations thereof.

In some embodiments, additional solutions are also included in the kit, such as alcohol, chondroitinase, endonuclease, and lipase, and endotoxin reducing solution, as previously described. The kits may also include a storage solution, preferably a non-aqueous solution such as mineral oil, for storing the resultant tissue matrix. Many embodiments further include instructions of use.

Any of the above-described kits may also include instructions for use of the kit. Moreover, some embodiments of the kits may also include at least one assay reagent for use in detecting regenerative repair and/or instructions for their use. These reagents are structured for use in detecting at least one of cellular infiltration, attachment, proliferation, differentiation, and synthesis of matrix materials appropriate to the regenerative repair of soft tissue defects. Methods of detection may include by conjugation of detectable labels or substrates, such as fluorescent compounds, enzymes, radioisotopes, heavy atoms, reporter genes, luminescent compounds, or antibodies against molecular components of the tissue matrix. As it would be understood by those skilled in the art, additional detection or labeling methodologies may be used in the kits provided.

Without further elaboration, it is believed that one skilled in the art can, using the preceding description, utilize the present invention to its fullest extent. The following examples are offered by way of illustration, not by way of limitation. While specific examples have been provided, the above description is illustrative and not restrictive. Anyone or more of the features of the previously described embodiments can be combined in any manner with one or more features of any other embodiments in the present invention. Furthermore, many variations of the invention will become apparent to those skilled in the art upon review of the specification.

All publications and patent documents cited in this application are incorporated by reference in pertinent part for all purposes to the same extent as if each individual publication or patent document were so individually denoted. By citation of various references in this document, Applicants do not admit any particular reference is "prior art" to their invention.

### EXAMPLES

The methods and compositions herein described and the related kits are further illustrated in the following examples, which are provided by way of illustration and are not intended to be limiting. It will be appreciated that variations in proportions and alternatives in elements of the components shown will be apparent to those skilled in the art and are within the scope of embodiments of the present invention. Theoretical aspects are presented with the understanding that Applicants do not seek to be bound by the theory presented. All parts or amounts, unless otherwise specified, are by weight.

### Example 1: Processing (A) of skin tissue in the production of a dermal matrix tissue.

### 1. Procedure

1.1 Preparation of Skin Connective Tissues
   1.1.1 If previously frozen in 1.5 M Sodium Chloride solution, remove wrapped skin package from freezer, place on orbital shaker at 60 RPM, and allow skin to thaw completely for 24 hours at 36°C ±1°C. If freshly recovered, proceed to processing as detailed below. If converting cryo-preserved skin to de-cellularized dermis, remove skin package from freezer and place in an incubator at 36°C ±1°C. After thawing, remove skin from sterile packaging and continue to section 1.2.
1.2 Inspection, Cleaning and Weighing of Tissues
   1.2.1 Using aseptic techniques, present skin tissues to the sterile field.
   1.2.2 Place each piece of skin with the epidermal side up on a cutting board.
   1.2.3 Inspect the skin tissues for holes, moles, warts, and tattoos and cut out these areas using a scalpel.
   1.2.4 Examine the tissues for hair and remove with forceps.
   1.2.5 Rinse cleaned tissues by placing into a basin containing sterile water.
   1.2.6 Using a scalpel, carefully cut a small, triangular notch approximately 0.5 cm below the upper left hand corner of the graft.
   1.2.7 Transfer tissues into an empty, sterile container and aseptically place on the pre-disinfected weighing scale. Determine and record the total weight (weight of the tissues and container) on the processing record.
   1.2.8 Aseptically transfer the tissues into a sterile plastic jar on the sterile field leaving the basin on the weighing scale. Determine and record the weight of the empty basin.
1.3 Rinsing of Tissues
   1.3.1 Based on the weight of the tissue determined in section 1.2.8, determine the volume of antibiotic-free normal saline solution to be utilized for one rinsing step.
   1.3.2 Add fresh, antibiotic-free normal saline solution (5 ml of saline per 1 gram of skin) into the sterile plastic jar, secure jar with its lid, and agitate aggressively for 15 seconds.
   1.3.3 Remove the lid and decant the solution.
   1.3.4 Repeat this rinsing step three (3) times.
      NOTE: USE THE PRESCRIBED VOLUME OF NEW SOLUTION FOR EACH RINSE.
   1.3.5 After the last rinse, decant solution.
1.4 Sodium Chloride Incubation
   1.4.1 Transfer the skin tissues into a new sterile plastic jar.
   1.4.2 Pour previously prepared 1.5M Sodium Chloride solution (5 ml per 1 gram of skin or solution volume determined in step 1.3.1) into the skin jar and secure the lid. Ensure all tissues, including surrogates, are completely immersed in the solution.
   1.4.3 Place the wrapped skin tissues on the orbital shaker/incubator unit. Close the lid of the unit.
   1.4.4 Set the speed of the orbital shaker to 60 RPM and allow tissues to rotate and incubate at 37°C for 18-24 hours.
1.5 Epidermis/Dermis Separation
   1.5.1 Remove the wrapped tissues from the incubator/shaker unit and aseptically unwrap the jar.
   1.5.2 Remove the lid of the jar and examine the tissues. There should be a noticeable separation of the epidermal and dermal skin layers.
   1.5.3 Transfer one skin tissue piece into a new sterile basin containing sterile water.
   1.5.4 Remove the epidermal layer.
   1.5.5 Transfer the dermal skin tissue into a new sterile basin containing sterile water.
   1.5.6 Continue this process until the epidermal layer has been removed from all the dermal skin tissue pieces.
   1.5.7 Re-weigh the dermal tissue to be utilized through the remaining processing step.
1.6 Rinse
   1.6.1 Transfer the skin tissues into a sterile plastic jar.
   1.6.2 Add fresh, sterile water (5 ml of saline per 1 gram of skin) into the sterile plastic jar, secure jar with its lid, and agitate aggressively for 15 seconds.
   1.6.3 Remove the lid and decant the solution.
   1.6.4 Repeat this rinsing step three (3) times.
   1.6.5 After the last rinse, decant solution.
   1.6.6 Place separate basins on the sterile field and label each one with the thickness of the tissue.
   1.6.7 Pour sterile normal saline in each basin.
   1.6.8 Cut tissues to specified dimensions using a scalpel and a ruler as a guide and place in the respective basin. Keep each basin separate when continuing each processing step. Place all trimmings of dermal skin tissue on the side of the sterile field until all grafts have been cut.
   1.6.9 After each graft is cut, place one cut graft with dermis side down and basement membrane facing up. Using a scalpel carefully cut a small, triangular notch approximately 0.5 cm below the upper left hand corner of the graft.
   1.6.10 Place cut graft with notch in the respective basin.
   1.6.11 Repeat the process until all grafts are cut with a notch.
1.7 Incubation in Detergent (Per Tissue Batch)
   1.7.1 Place the dermal skin tissue grafts into a new sterile plastic container with lid.
   1.7.2 Pour 0.5% BRIJ 35 solution (Poly (oxyethylene) (23) lauryl ether, 10% aqueous solution, proteomic grade) (5 ml of solution per 1 gram of skin) (G-Biosciences, Cat.#:DG004) into the container and secure the lid. Ensure all tissues are completely immersed in the solution.
   1.7.3 Place the wrapped skin tissues on the orbital shaker/incubator unit. Close the lid of the unit.
   1.7.4 Set the speed of the orbital shaker to 60 RPM and allow tissues to rotate and incubate at 37°C for 18-24 hours.
1.8 Prepare the solution (0.1% Peracetic Acid Solution).
1.9 Rinse
   1.9.1 After incubation, remove the wrapped tissues from the incubator/shaker unit and aseptically unwrap the jar.
   1.9.2 Remove the lid of the jar and decant the 0.5% BRIJ 35 solution.
   1.9.3 Add sterile water (5 ml solution per 1 gram of skin) to the container and agitate aggressively for 30 seconds.
   1.9.4 Decant solution.
   1.9.5 Repeat the process eight (8) times.
1.10 Disinfection
   1.10.1 Transfer the dermal skin tissue grafts into a new sterile plastic container with lid.
   1.10.2 Pour 0.1% peracetic acid solution (5 ml per 1 gram of skin) into the container and secure the lid. Ensure all tissues are completely immersed in the solution
   1.10.3 Place the wrapped skin tissues on the orbital shaker/incubator unit. Close the lid of the unit.
   1.10.4 Set the speed of the orbital shaker to 60 RPM and allow tissues to rotate and incubate at 37°C for 4 hours.
1.11 Rinse
   1.11.1 After incubation, remove the wrapped tissues from the incubator/shaker unit and aseptically unwrap the jar.
   1.11.2 Remove the lid of the jar and decant the 0.1% Peracetic acid solution.
   1.11.3 Add sterile water (5 ml solution per 1 gram of skin) to the container and agitate aggressively for 30 seconds.
   1.11.4 Decant solution.
   1.11.5 Repeat the process eight (8) times.
1.12 Lyophilization
   1.12.1 Load the tissues in the freeze dryer.
   1.12.2 Perform the Lyophilization process in accordance with *applicable Lyophilization* parameters - *Operation of the VirTis Freeze Dryers.*
   1.12.3 Lyophilize the tissues for 2 to 10 days.

### Example 2: Processing (B) of skin for the production of dermal matrix.

### 2 Procedure

2.1 Preparation of Skin Connective Tissues
   2.1.1 If previously frozen in 1.5M Sodium Chloride solution, remove wrapped skin package from freezer, place on orbital shaker at 60 RPM, and allow skin to thaw completely for 24 hours at 36°C ±1°C. DO NOT UNSEAL THE PACKAGING. Skip to section 2.2
   2.1.2 If freshly recovered, proceed to processing as detailed below. Continue to section 2.2
   2.1.3 If converting cryo-preserved skin to de-cellularized dermis, remove skin package from freezer and place in an incubator at 36°C ±1°C. After thawing, remove skin from sterile packaging and continue to section 2.2.
2.2 Inspection, Cleaning and Weighing of Tissues
   2.2.1 Using aseptic technique; present skin tissues to the sterile field.
   2.2.2 Place each piece of skin with the epidermal side up on a cutting board.
   2.2.3 Inspect the skin tissues for holes, moles, warts, and tattoos and cut out these areas using a scalpel.
   2.2.4 Examine the tissues for hair and remove with forceps.
   2.2.5 Rinse cleaned tissues by placing into a basin containing sterile water.
   2.2.6 Using a scalpel carefully cut a small, triangular notch approximately 0.5 cm below the upper left hand corner of the graft.
   2.2.7 Transfer tissues into an empty, sterile container and aseptically place on the pre-disinfected weighing scale. Determine and record the total weight (weight of the tissues and container) on the processing record.
   2.2.8 Aseptically transfer the tissues into a sterile plastic jar on the sterile field leaving the basin on the weighing scale. Determine and record the weight of the empty basin.
2.3 Rinsing of Tissues
   2.3.1 Based on the weight of the tissue determined in section 2.2, determine the volume of antibiotic-free normal saline solution to be utilized for one rinsing step.
   2.3.2 Add fresh, antibiotic-free normal saline solution (5 ml of saline per 1 gram of skin) into the sterile plastic jar, secure jar with its lid, and agitate aggressively for 15 seconds.
   2.3.3 Remove the lid and decant the solution.
   2.3.4 Repeat this rinsing step three (3) times.
      NOTE: USE THE PRESCRIBED VOLUME OF NEW SOLUTION FOR EACH RINSE.
   2.3.5 After the last rinse, decant solution.
2.4 Sodium Chloride Incubation
   2.4.1 Transfer the skin tissues into a new sterile plastic jar.
   2.4.2 Pour previously prepared, 1.5M Sodium Chloride solution (5 ml per 1 gram of skin or solution volume determined in step 2.3.1) into the skin jar and secure the lid. Ensure all tissues, including surrogates, are completely immersed in the solution.
   2.4.3 Place the wrapped skin tissues on the orbital shaker/incubator unit. Close the lid of the unit.
   2.4.4 Set the speed of the orbital shaker to 60 RPM and allow tissues to rotate and incubate at 37°C for 18-24 hours.
2.5 Epidermis/Dermis Separation
   2.5.1 Remove the wrapped tissues from the incubator/shaker unit and aseptically unwrap the jar.
   2.5.2 Remove the lid of the jar and examine the tissues. There should be a noticeable separation of the epidermal and dermal skin layers.
   2.5.3 Transfer one skin tissue piece into a new sterile basin containing sterile water.
   2.5.4 Remove the epidermal layer.
   2.5.5 Transfer the dermal skin tissue into a new sterile basin containing sterile water.
   2.5.6 Continue this process until the epidermal layer has been removed from all the dermal skin tissue pieces.
   2.5.7 Re-weigh the dermal tissue to be utilized through the remaining processing step.
2.6 Rinse
   2.6.1 Transfer the skin tissues into a sterile plastic jar.
   2.6.2 Add fresh, sterile water (5 ml of saline per 1 gram of skin) into the sterile plastic jar, secure jar with its lid, and agitate aggressively for 15 seconds.
   2.6.3 Remove the lid and decant the solution.
   2.6.4 Repeat this rinsing step three (3) times.
   2.6.5 After the last rinse, decant solution.
   2.6.6 Place separate basins on the sterile field and label each one with the thickness of the tissue.
   2.6.7 Pour sterile normal saline in each basin.
   2.6.8 Cut tissues to specified dimensions using a scalpel and a ruler as a guide and place in the respective basin. Keep each basin separate when continuing each processing step. Place all trimmings of dermal skin tissue on the side of the sterile field until all grafts have been cut.
   2.6.9 After each graft is cut, place one cut graft with dermis side down and basement membrane facing up. Using a scalpel carefully cut a small, triangular notch approximately 0.5 cm below the upper left hand corner of the graft.
   2.6.10 Place cut graft with notch in the respective basin.
   2.6.11 Repeat the process until all grafts are cut with a notch.
2.7 Incubation in Detergent (Per Tissue Batch)
   2.7.1 Place the dermal skin tissue grafts into a new sterile plastic container with lid.
   2.7.2 Pour 0.5% BRIJ 35 solution (5 ml of solution per 1 gram of skin) into the container and secure the lid. Ensure all tissues are completely immersed in the solution.
   2.7.3 Place the wrapped skin tissues on the orbital shaker/incubator unit. Close the lid of the unit.
   2.7.4 Set the speed of the orbital shaker to 60 RPM and allow tissues to rotate and incubate at 37°C for 18-24 hours.
2.8 Incubate in Chondroitinase ABC
   2.8.1 Place the dermal skin tissue grafts into a new sterile plastic container with lid
   2.8.2 Pour saline solution containing 100 Units of Chondroitinase ABC (5 ml of solution/ 1 gram of tissue) into the container and secure the lid. Ensure all tissues are completely immersed in the solution.
   2.8.3 Place the wrapped tissue on the orbital shaker and incubate unit. Close the lid of the unit.
   2.8.4 Set the speed of the orbital shaker to 60 RPM and allow tissues to rotate and incubate at 37°C for 4-6 hours.
2.9 Prepare the solution (0.1% Peracetic Acid Solution) to be utilized on Day 3.
2.10 Rinse
   2.10.1 After incubation, remove the wrapped tissues from the incubator/shaker unit and aseptically unwrap the jar.
   2.10.2 Remove the lid of the jar and decant the 0.5% BRIJ 35 solution.
   2.10.3 Add sterile water (5 ml solution per 1 gram of skin) to the container and agitate aggressively for 30 seconds.
   2.10.4 Decant solution.
   2.10.5 Repeat the process eight (8) times.
2.11 Disinfection
   2.11.1 Transfer the dermal skin tissue grafts into a new sterile plastic container with lid.
   2.11.2 Pour 0.1% Peracetic acid solution (5 ml per 1 gram of skin) into the container and secure the lid. Ensure all tissues are completely immersed in the solution
   2.11.3 Place the wrapped skin tissues on the orbital shaker/incubator unit. Close the lid of the unit.
   2.11.4 Set the speed of the orbital shaker to 60 RPM and allow tissues to rotate and incubate at 37°C for 4 hours.
2.12 Rinse
   2.12.1 After incubation, remove the wrapped tissues from the incubator/shaker unit and aseptically unwrap the jar.
   2.12.2 Remove the lid of the jar and decant the 0.1% Peracetic acid solution.
   2.12.3 Add sterile water (5 ml solution per 1 gram of skin) to the container and agitate aggressively for 30 seconds.
   2.12.4 Decant solution.
   2.12.5 Repeat the process eight (8) times.
2.13 Lyophilization
   2.13.1 Load the tissues in the freeze dryer.
   2.13.2 Perform the Lyophilization process in accordance to *applicable Lyophilization* parameters - *Operation of the VirTis Freeze Dryers.*
   2.13.3 Lyophilize the tissues for 2 to 10 days.

### Example 3: Preparation (A) of fascia for connective tissue matrix

### Preparation of Fascia Connective Tissues

2.1 Rinsing of Tissues
   Based on the weight of the tissue determined in section 2.2, determine the volume of antibiotic-free normal saline solution to be utilized for one rinsing step.
   2.1.1 Add fresh, antibiotic-free normal saline solution (5 ml of saline per 1 gram of fascia) into the sterile plastic jar, secure jar with its lid, and agitate aggressively for 15 seconds.
   2.1.2 Remove the lid and decant the solution.
   2.1.3 Repeat this rinsing step three (3) times.
      NOTE: USE THE PRESCRIBED VOLUME OF NEW SOLUTION FOR EACH RINSE.
   2.1.4 After the last rinse, decant solution.
2.2 Rinse
   2.2.1 Transfer the fascia tissues into a sterile plastic jar.
   2.2.2 Add fresh, sterile water (5 ml of saline per 1 gram of fascia) into the sterile plastic jar, secure jar with its lid, and agitate aggressively for 15 seconds.
   2.2.3 Remove the lid and decant the solution.
   2.2.4 Repeat this rinsing step three (3) times.
   2.2.5 After the last rinse, decant solution.
   2.2.6 Place separate basins on the sterile field and label each one with the thickness of the tissue.
   2.2.7 Pour sterile normal saline in each basin.
   2.2.8 Cut tissues to specified dimensions using a scalpel and a ruler as a guide and place in the respective basin. Keep each basin separate when continuing each processing step. Place all trimmings of fascia tissue on the side of the sterile field until all grafts have been cut.
   2.2.9 After each graft is cut, place one cut graft with inferior side down and the superior membrane facing up. Using a scalpel carefully cut a small, triangular notch approximately 0.5 cm below the upper left hand corner of the graft.
   2.2.10 Place cut graft with notch in the respective basin.
   2.2.11 Repeat the process until all grafts are cut with a notch.
2.3 Incubation in Detergent (Per Tissue Batch)
   2.3.1 Place the fascia tissue grafts into a new sterile plastic container with lid.
   2.3.2 Pour 0.5% BRIJ 35 solution (5 ml of solution per 1 gram of tissue) into the container and secure the lid. Ensure all tissues are completely immersed in the solution.
   2.3.3 Place the wrapped tissues on the orbital shaker/incubator unit. Close the lid of the unit.
   2.3.4 Set the speed of the orbital shaker to 60 RPM and allow tissues to rotate and incubate at 37°C for 18-24 hours.
2.4 Incubate in Chondroitinase ABC
   2.4.1 Place the tissue grafts into a new sterile plastic container with lid
   2.4.2 Pour saline solution containing 50 Units of Chondroitinase ABC (5 ml of solution/ 1 gram of tissue) into the container and secure the lid. Ensure all tissues are completely immersed in the solution.
   2.4.3 Place the wrapped tissue on the orbital shaker and incubate unit. Close the lid of the unit.
   2.4.4 Set the speed of the orbital shaker to 60 RPM and allow tissues to rotate and incubate at 37°C for 4-6 hours.
2.5 Prepare the solution (0.1% Peracetic Acid Solution) to be utilized on Day 3.
2.6 Rinse
   2.6.1 After incubation, remove the wrapped tissues from the incubator/shaker unit and aseptically unwrap the jar.
   2.6.2 Remove the lid of the jar and decant the chondroitinase solution.
   2.6.3 Add sterile water (5 ml solution per 1 gram of tissue) to the container and agitate aggressively for 30 seconds.
   2.6.4 Decant solution.
   2.6.5 Repeat the process eight (8) times.
2.7 Disinfection
   2.7.1 Transfer the tissue grafts into a new sterile plastic container with lid.
   2.7.2 Pour 0.1% Peracetic acid solution (5 ml per 1 gram of tissue) into the container and secure the lid. Ensure all tissues are completely immersed in the solution
   2.7.3 Place the wrapped tissues on the orbital shaker/incubator unit. Close the lid of the unit.
   2.7.4 Set the speed of the orbital shaker to 60 RPM and allow tissues to rotate and incubate at 37°C for 4 hours.
2.8 Rinse
   2.8.1 After incubation, remove the wrapped tissues from the incubator/shaker unit and aseptically unwrap the jar.
   2.8.2 Remove the lid of the jar and decant the 0.1% Peracetic acid solution.
   2.8.3 Add sterile water (5 ml solution per 1 gram of tissue) to the container and agitate aggressively for 30 seconds.
   2.8.4 Decant solution.
   2.8.5 Repeat the process eight (8) times.
2.9 Lyophilization
   2.9.1 Load the tissues in the freeze dryer.
   2.9.2 Perform the Lyophilization process in accordance to *applicable Lyophilization* parameters - *Operation of the VirTis Freeze Dryers.*
   2.9.3 Lyophilize the tissues for 2 to 10 days.

### Example 4: Preparation (B) of fascia connective tissue matrix 2.10 Preparation of Fascia Connective Tissues

2.11 Rinsing of Tissues
   2.11.1 Based on the weight of the tissue determined in section 2.2, determine the volume of antibiotic-free normal saline solution to be utilized for one rinsing step.
   2.11.2 Add fresh, antibiotic-free normal saline solution (5 ml of saline per 1 gram of fascia) into the sterile plastic jar, secure jar with its lid, and agitate aggressively for 15 seconds.
   2.11.3 Remove the lid and decant the solution.
   2.11.4 Repeat this rinsing step three (3) times.
   2.11.5 After the last rinse, decant solution.
2.12 Rinse
   2.12.1 Transfer the fascia tissues into a sterile plastic jar.
   2.12.2 Add fresh, sterile water (5 ml of saline per 1 gram of fascia) into the sterile plastic jar, secure jar with its lid, and agitate aggressively for 15 seconds.
   2.12.3 Remove the lid and decant the solution.
   2.12.4 Repeat this rinsing step three (3) times.
   2.12.5 After the last rinse, decant solution.
   2.12.6 Place separate basins on the sterile field and label each one with the thickness of the tissue.
   2.12.7 Pour sterile normal saline in each basin.
   2.12.8 Cut tissues to specified dimensions using a scalpel and a ruler as a guide and place in the respective basin. Keep each basin separate when continuing each processing step. Place all trimmings of fascia tissue on the side of the sterile field until all grafts have been cut.
   2.12.9 After each graft is cut, place one cut graft with inferior side down and the superior membrane facing up. Using a scalpel carefully cut a small, triangular notch approximately 0.5 cm below the upper left hand corner of the graft.
   2.12.10 Place cut graft with notch in the respective basin.
   2.12.11 Repeat the process until all grafts are cut with a notch.
2.13 Incubation in Detergent (Per Tissue Batch)
   2.13.1 Place the fascia tissue grafts into a new sterile plastic container with lid.
   2.13.2 Pour 0.5% BRIJ 35 solution (5 ml of solution per 1 gram of tissue) into the container and secure the lid. Ensure all tissues are completely immersed in the solution.
   2.13.3 Place the wrapped tissues on the orbital shaker/incubator unit. Close the lid of the unit.
   2.13.4 Set the speed of the orbital shaker to 60 RPM and allow tissues to rotate and incubate at 37°C for 18-24 hours.
2.14 Prepare the solution (0.1% Peracetic Acid Solution) to be utilized.
2.15 Rinse
   2.15.1 After incubation, remove the wrapped tissues from the incubator/shaker unit and aseptically unwrap the jar.
   2.15.2 Remove the lid of the jar and decant the detergent solution.
   2.15.3 Add sterile water (5 ml solution per 1 gram of tissue) to the container and agitate aggressively for 30 seconds.
   2.15.4 Decant solution.
   2.15.5 Repeat the process eight (8) times.
2.16 Disinfection
   2.16.1 Transfer the tissue grafts into a new sterile plastic container with lid.
   2.16.2 Pour 0.1% Peracetic acid solution (5 ml per 1 gram of tissue) into the container and secure the lid. Ensure all tissues are completely immersed in the solution
   2.16.3 Place the wrapped tissues on the orbital shaker/incubator unit. Close the lid of the unit.
   2.16.4 Set the speed of the orbital shaker to 60 RPM and allow tissues to rotate and incubate at 37°C for 4 hours.
2.17 Rinse
   2.17.1 After incubation, remove the wrapped tissues from the incubator/shaker unit and aseptically unwrap the jar.
   2.17.2 Remove the lid of the jar and decant the 0.1% Peracetic acid solution.
   2.17.3 Add sterile water (5 ml solution per 1 gram of tissue) to the container and agitate aggressively for 30 seconds.
   2.17.4 Decant solution.
   2.17.5 Repeat the process eight (8) times.
2.18 Lyophilization
   2.18.1 Load the tissues in the freeze dryer.
   2.18.2 Perform the Lyophilization process in accordance to *applicable Lyophilization* parameters - *Operation of the VirTis Freeze Dryers.*
   2.18.3 Lyophilize the tissues for 2 to 10 days.

### Example 5: Preparation of pericardial tissue matrix

2.19 Rinsing of Tissues
   2.19.1 Based on the weight of the tissue determined in section 2.2, determine the volume of antibiotic-free normal saline solution to be utilized for one rinsing step.
   2.19.2 Add fresh, antibiotic-free normal saline solution (5 ml of saline per 1 gram of tissue) into the sterile plastic jar, secure jar with its lid, and agitate aggressively for 15 seconds.
   2.19.3 Remove the lid and decant the solution.
   2.19.4 Repeat this rinsing step three (3) times.
   2.19.5 After the last rinse, decant solution.
2.20 Rinse
   2.20.1 Transfer the tissues into a sterile plastic jar.
   2.20.2 Add fresh, sterile water (5 ml of saline per 1 gram of tissue) into the sterile plastic jar, secure jar with its lid, and agitate aggressively for 15 seconds.
   2.20.3 Remove the lid and decant the solution.
   2.20.4 Repeat this rinsing step three (3) times.
   2.20.5 After the last rinse, decant solution.
   2.20.6 Place separate basins on the sterile field and label each one with the thickness of the tissue.
   2.20.7 Pour sterile normal saline in each basin.
   2.20.8 Cut tissues to specified dimensions using a scalpel and a ruler as a guide and place in the respective basin. Keep each basin separate when continuing each processing step. Place all trimmings of pericardial tissue on the side of the sterile field until all grafts have been cut.
   2.20.9 After each graft is cut, place one cut graft with inferior side down and the superior membrane facing up. Using a scalpel carefully cut a small, triangular notch approximately 0.5 cm below the upper left hand corner of the graft.
   2.20.10 Place cut graft with notch in the respective basin.
   2.20.11 Repeat the process until all grafts are cut with a notch.
2.21 Incubation in Detergent (Per Tissue Batch)
   2.21.1 Place the tissue grafts into a new sterile plastic container with lid.
   2.21.2 Pour 0.5% BRIJ 35 solution (5 ml of solution per 1 gram of tissue) into the container and secure the lid. Ensure all tissues are completely immersed in the solution.
   2.21.3 Place the wrapped tissues on the orbital shaker/incubator unit. Close the lid of the unit.
   2.21.4 Set the speed of the orbital shaker to 60 RPM and allow tissues to rotate and incubate at 37°C for 18-24 hours.
2.22 Incubate in Chondroitinase ABC
   2.22.1 Place the tissue grafts into a new sterile plastic container with lid.
   2.22.2 Pour saline solution containing 150 Units of Chondroitinase ABC (5 ml of solution/ 1 gram of tissue) into the container and secure the lid. Ensure all tissues are completely immersed in the solution.
   2.22.3 Place the wrapped tissue on the orbital shaker and incubate unit. Close the lid of the unit.
   2.22.4 Set the speed of the orbital shaker to 60 RPM and allow tissues to rotate and incubate at 37°C for 4-6 hours.
2.23 Prepare the solution (0.1% Peracetic Acid Solution).
2.24 Rinse
   2.24.1 After incubation, remove the wrapped tissues from the incubator/shaker unit and aseptically unwrap the jar.
   2.24.2 Remove the lid of the jar and decant the chondroitinase solution.
   2.24.3 Add sterile water (5 ml solution per 1 gram of tissue) to the container and agitate aggressively for 30 seconds.
   2.24.4 Decant solution.
   2.24.5 Repeat the process eight (8) times.
2.25 Disinfection
   2.25.1 Transfer the tissue grafts into a new sterile plastic container with lid.
   2.25.2 Pour 0.1% Peracetic acid solution (5 ml per 1 gram of tissue) into the container and secure the lid. Ensure all tissues are completely immersed in the solution.
   2.25.3 Place the wrapped tissues on the orbital shaker/incubator unit. Close the lid of the unit.
   2.25.4 Set the speed of the orbital shaker to 60 RPM and allow tissues to rotate and incubate at 37°C for 4 hours.
2.26 Rinse
   2.26.1 After incubation, remove the wrapped tissues from the incubator/shaker unit and aseptically unwrap the jar.
   2.26.2 Remove the lid of the jar and decant the 0.1% Peracetic acid solution.
   2.26.3 Add sterile water (5 ml solution per 1 gram of tissue) to the container and agitate aggressively for 30 seconds.
   2.26.4 Decant solution.
   2.26.5 Repeat the process eight (8) times.
2.27 Lyophilization
   2.27.1 Load the tissues in the freeze dryer.
   2.27.2 Perform the Lyophilization process in accordance to *applicable Lyophilization* parameters - *Operation of the VirTis Freeze Dryers.*
   2.27.3 Lyophilize the tissues for 2 to 10 days.

### Example 6: Cellular infiltration onto and into processed dermal matrix.

Freeze-dried dermis samples were prepared by the processing unit using a 10-mm biopsy punch (Acuderm Inc., Ft. Lauderdale). Biocompatibility was assessed by direct contact of L929 and MIAMI cells (a proprietary cell line as identified and described in U.S. Patent No. 7,807,458) with the dermal matrix samples for various periods of time. Qualitative histology assessment was performed and results collected and documented.

It was hypothesized that tissue samples lacking agents which may be cytotoxic to mammalian cells and which present a matrix structure which at the molecular and macroscopic level provide an environment which is suitable and appropriate for cellular attachment and proliferation were deemed to be biocompatible for cellular attachment and proliferation. When such tissues are tested in this manner and found to be biocompatible there is a strong presumption that such tissue matrices will serve in a regenerative capacity when used clinically in the treatment of pathologies.

### Materials and methods:

*All procedures were carried out in a laminar flow hood designed to provide the sterile working environment. Sterile tools and containers were used.*

### Experimental procedure:

1) L-929 cells or MIAMI cells were expanded and used before reaching confluency.
2) Each dermal matrix sample was transferred (basement membrane facing down) into individual wells of 24 well plates (Ultra Low Attachment surface, Corning, #3473) containing 500 µL of L-929 or MIAMI cell expansion media.
3) 10x10³ or 200x10³ L929 or MIAMI cells were harvested and seeded directly onto the surface of the dermal matrix tissue samples in the wells (final volume of media/wells: 1000 pL). Dermal matrix samples without cells were used as negative controls.
4) Plates were incubated at 37°C and 5% CO₂ on a rocker (Rocker II, #260350, Boekel Scientific, 15 oscillations/min) and samples of dermal matrix tissue were collected and placed into formalin (4% CH₂O, 1% methanol) for histology preparations at different days to provide an understanding of the behavior of cells after short, medium and long term contact with the dermis.
5) Paraffin embedding, slicing and Hematoxylin/Eosin staining of the samples were performed according to standard histological methodologies.

### Results:

Histology preparations were examined and digital images taken of representative sections with the objective of illustrating the adherence of either L-929 or MIAMI cells onto the surfaces of the dermal matrix tissue samplesm as well as any evidence of cellular proliferation.

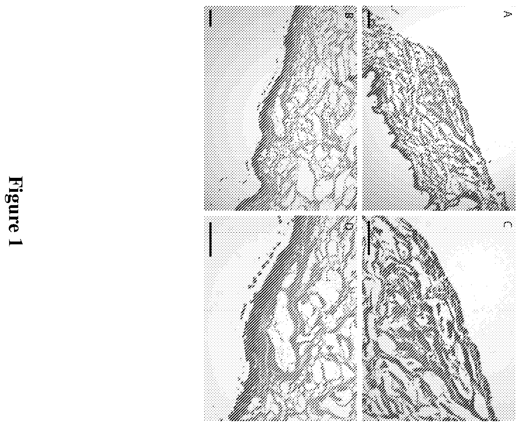

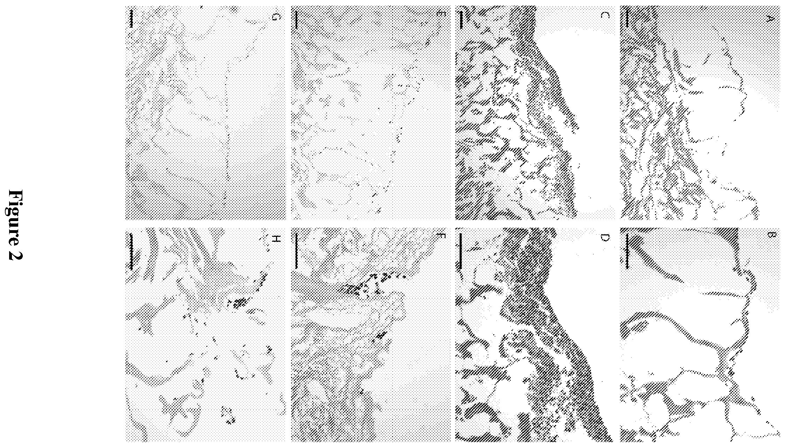

As illustrated in Figures 1 and 2, the dermal matrix easily supported cellular attachment and cellular proliferation of L-929 cells (Figure 1 from a first donor, and Figure 2 from a second donor). Plating of only a low density of cells (10000 cells/sampe) as depicted in Figure 1 seems to show that L-929 cells proliferated in contact of the dermis, with only little cells observed at early time points compared to the amount observed after 36 days.

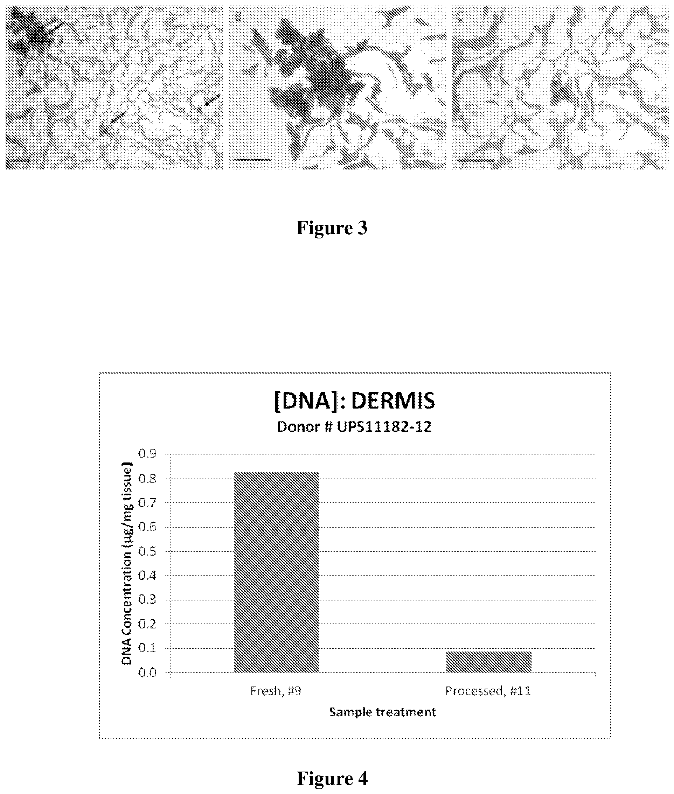

Similarly, as illustrated in Figure 3, the less robust MIAMI cells also survived in contact of the dermal matrix.

Both the cell types were seen to infiltrate the tissue, thereby, indicating the dermal matrix was biocompatible and readily supports cell growth in an *in vitro* situation.

### Example 7A: Reduction in extractable DNA in dermal matrix samples.

DNA extraction and quantification from dermal matrix was used to reflect the efficiency of the decellularization process.

### Materials and Methods:

DNA was quantified in:
- Fresh dermis, not processed
- Freeze dried, washed dermis
*Note: all materials coming in contact with the samples were sterile to avoid exogenous DNA contamination. The DNA extraction kit (QIAamp DNA Mini, Qiagen, #51304) was used according to the manufacturer instructions, with modifications as described in the following procedure. Every step related to the kit was performed at room temperature.*
1) Samples of dermis were weighed and approximately 10 mg were transferred to sterile 2 ml microcentrifuge tubes (eppendorfs®). The exact weight was recorded in the laboratory notebook. For samples that were not washed 1.5 mg was used.
2) The volume of tissue lysis buffer (Buffer ATL) was adjusted per sample according to its weight (360 µl of lysis buffer/ 5 mg, and 360 µl/1.5 mg).
3) The volume of proteinase K was adjusted per sample according to its weight (40 µl proteinase K /5 mg of sample and same volume for 1.5 mg) was added and mixed by pulse-vortexing for 15 sec.
4) Samples were incubated at 56°C for 20 hrs until fully lysed.
5) 400 µl of each sample was placed in a new eppendorf tube and 8 µl RNase A (100 mg/ml) was added, mixed by pulse-vortexing for 15 sec, and incubated for 2 min at room temperature.
6) 400 µl lysis buffer (Buffer AL) was added and mixed by pulse-vortexing for 15 sec.
7) Samples were incubated at 70°C for 10 min.
8) 400 µl ethanol (100%) was added and mixed by pulse-vortexing for 15 sec.
9) Half of the lysates (600 µl) were transferred to the QIAamp MinElute columns.
10) Columns were centrifuged at 6000g for 1 min.
11) Columns were placed in clean 2 ml collection tubes. Steps 9, 10 and 11 were repeated with the other half of the lysate in the same column.
12) 500 µl washing buffer (Buffer AW1) was added to the columns and centrifuged at 6000g for 1 min.
13) Columns were placed in clean 2 ml collection tubes.
14) Steps 12 and 13 were repeated.
15) 500 µl washing buffer (Buffer AW2) was added and incubated for 2 min before centrifugation at 18,000g for 1 min.
16) Columns were placed in clean 2 ml collection tubes.
17) 500 µl washing buffer (Buffer AW2) was added, and incubated for 2 minutes before centrifugation at 18,000g for 3 min.
18) Columns were placed in clean 2 ml collection tubes.
19) Column was centrifuged at 18,000g for 1 min to dry the membrane.
20) Columns were placed in clean 2 ml collection tubes.
21) 100 µl TE buffer was applied on the center of the column and incubated at room temperature (15-25°C) for 5 min.
22) Columns were centrifuged at 6000g for 1 min.
23) Columns were placed in another clean microcentrifuge tube.
24) Steps 21-23 were repeated twice.
25) The samples were vortexed and then the DNA concentration was determined using a Nanodrop ND-1000 spectrophotometer (Thermo Fisher Scientific). The instrument was initialized using water and blank using TE buffer.

### Results:

The amount of DNA extracted from each dermal sample was determined, giving the results shown below:

**Table 1**

| **Donors/SCI #** | **Processing Conditions** | **Washing Conditions** | **Starting Weight (mg)** | **Final Weight (mg)** | **µg DNA/mg of product** |
|---|---|---|---|---|---|
| UPS11182-12 | Dermis. Not processed, Control | Not washed | 7.8 | 1.5 | 0.83 |
| UPS111140024 | Dermis. Not processed | Not washed | 10.45 | 1.5 | 0.66 |
| UPS11182-12 | Dermis. Not micronized, FD | Washed | 11.67 | 10 | 0.085 |

**Table 2**

| **Donor ID** | **Sample Type** | **µg DNA/mg tissue** |
|---|---|---|
| UPS11182-12 | Dermis,fresh,unprocessed | 0.827 |
| UPS11182-12 | Dermis,processed (SOP 25-009 02) | 0.085 |

### Conclusion:

Two fresh, non-processed dermis samples from different donors were analyzed. Both had similar DNA contents (0.83 & 0.66 µg DNA/mg tissue). Samples of dermis were processed according to the process of the present invention, such as described above in Example 1. This dermis sample analyzed had 90% less DNA than the fresh, non-processed sample of the same donor (0.085 & 0.83 µg DNA/mg tissue, respectively). This is shown in Tables 1 and 2 above, and depicted in the graph of Figure 4.

### Example 7B: Effects of detergent and peracetic acid in reducing extractable DNA in dermal matrix samples.

Dermal matrix samples were prepared by the processing unit using a 10-mm biopsy punch (Acuderm inc., Ft. Lauderdale). DNA is quantified in samples taken at pre-detergent processing, post-detergent processing, and post-peracetic acid processing steps. Several donors are analyzed during these 2 steps. Five samples from each single donor are used to average the results:

### DNA extraction procedure:

1) Samples collected from the processing room were flash-frozen in liquid nitrogen to prevent DNA degradation.
2) Slowly thawed the samples on ice
3) Excess water was removed from the samples by placing them on sterile blotting paper for 2-3 minutes.
   Note: all materials coming in contact with the samples are sterile to avoid exogenous DNA contamination.
4) Sub-samples of approximate weight 12.5 mg were excised from every sample and transferred to sterile microcentrifuge tubes (Eppendorf®). The exact weight is recorded in the laboratory notebook.
   *Note: a DNA extraction kit (QIAamp DNA Mini, Qiagen, #51304) was used according to the manufacturer instructions, with modifications as described in the following. All steps were performed at room temperature.*
5) Add 180 µL of tissue lysis buffer (Buffer ATL) to each sample.
6) Fully dissociate the samples by use of micro-scissors.
7) Add 40 µL proteinase K and mix by pulse-vortexing for 15 seconds.
8) Incubate at 56°C overnight to fully lyse the sample.
10) Add 4 µL RNase A (100 mg/ml), mix by pulse-vortexing for 15 seconds, and incubate for 2 minutes at room temperature.
9) Add 200 µL lysis buffer (Buffer AL) and mix by pulse-vortexing for 15 s.
11) Incubate at 70°C for 10 minutes.
12) Add 200 µL ethanol (100%) and mix by pulse-vortexing for 15 seconds.
13) Carefully transfer the entire lysate from to the QIAamp MinElute column.
14) Centrifuge at 6000g for 1 min.
15) Place the QIAamp MinElute column in a clean 2 mL collection tube, and discard the collection tube containing the flow-through.
16) Add 500 µL washing buffer (Buffer AW1) and centrifuge at 6000g for 1 minute.
17) Place the QIAamp MinElute column in a clean 2 mL collection tube, and discard the collection tube containing the flow-through.
18) Add 500 µL washing buffer (Buffer AW2) and centrifuge at 20000g for 3 minutes.
19) Place the QIAamp MinElute column in a clean 2 mL collection tube, and discard the collection tube containing the flow-through.
20) Centrifuge at 20,000 X g for 1 minute to dry the membrane.
21) Place the QIAamp MinElute column in a clean microcentrifuge tube and discard the collection tube containing the flow-through.
22) Apply 200 µL elution buffer (Buffer AE) and incubate at room temperature (15-25°C) for 1 minute.
23) Centrifuge at 6000 X g for 1 minute.
24) Place the QIAamp MinElute column in another clean microcentrifuge tube and repeat step 22-23.
25) Quantify the double-strand DNA concentration in the samples using a Nanodrop ND-1000 spectrophotometer (Thermo Fisher Scientific). Initialize the instrument using water and blank using elution buffer (buffer AE).

### Results:

The amount of DNA present per weight of one sample is calculated from the average amount of the 2 samples of DNA collected in steps 23 and 24 of the protocol. For a single step, the results obtained in five samples are then averaged to give the results presented in the table below:

**Table 3**

| **Donors** | **Steps** | **ng of DNA/mg of sample (n=5)** | **Standard deviation** |
|---|---|---|---|
| UPR 9611 | Pre-BRIJ | 304.3 | 89.7 |
| | BRIJ 0.2% Post-PAA | 63.0 | 44.6 |
| | BRIJ 0.4% Post-PAA | 92.7 | 46.4 |
| UPS9238 | Pre-BRIJ | 444.5 | 90.8 |
| | BRIJ 0.2% Post-PAA | 114.7 | 34.9 |
| | BRIJ 0.4% Post-PAA | 239.8 | 16.5 |
| UPR9656 | Pre-BRIJ | 242.4 | 29.5 |
| | BRIJ 0.5% Post-PAA | 125.1 | 28.8 |

### Conclusion:

On average, experiments performed on 3 donors (5 samples per donor at each step), revealed that **63±15%** of DNA are removed between the Pre-BRIJ steps and the Post-PAA steps.

### Example 8: Inhibition of cellular proliferation and cellular activity of non-processed fascia tissue and an example of elements present in non-processed tissue which may inhibit cellular attachment and proliferation to tissues under in vivo and/or in vitro conditions.

Frozen fascia tissue samples were prepared by the processing unit for use in this study. The fascia tissue samples were only debrided of extrinsic blood elements and were not processed according to the present invention. To assess the cellular inhibition properties of extracts of these tissues, compounds present in the final products were extracted during 24 hours following ISO 10993-5 and ISO 10993-12 guidelines. The extraction media, containing potential inhibitory compounds, were then applied to fibroblast cells in culture (L-929 cell line ATCC #CCL-1). A quantitative assessment of healthy cell density was performed using the CyQUANT® Direct Cell Proliferation Assay while qualitative assessments of cell morphology were performed by microscopic observation.

CyQUANT® Direct Cell Proliferation Assay was chosen for its ability to provide a measure of viable cell density in a single step experiment. Indeed, it combines a DNA-binding dye and a background suppression reagent. The DNA-binding dye is a live-cell permeable reagent that mainly concentrates in the nucleus of metabolically viable mammalian cells, while the suppression dye is impermeable to living (viable) cells and quenches the fluorescence of DNA-binding dye in cells with compromised cell membrane. The combination of these two components results in an assay based on both DNA content and cell membrane integrity.

It was hypothesized that the assessment of cytotoxicity of extracts of materials involves an extraction of potentially toxic agents from materials and subsequent application of these extracts onto a population of metabolically viable mammalian cells. The CyQUANT® Direct Cell Proliferation Assay utilizes a dye which binds to nucleic acids and will bind with DNA of both living and metabolically non-living cells. However, the assay also employs a background suppression reagent which will suppress fluorescence of the dye. In that the background suppression reagent will not penetrate into the interior of a viable cell, any viable cell in the population of cells will fluoresce, but non-living cells will not fluoresce due to the quenching action of the background suppression reagent. This assay is thus a useful tool in determining the percentages of viable versus non-viable cells in a population of cells and any extract of a material which contains extractable agents toxic to a mammalian cell will alter this percentage of viable to non-viable cells and can thus be used to quantitatively determine toxicity of extracts.

### Materials and methods:

All procedures were carried out in an aseptic working environment. The following materials and conditions were adapted from the recommendations described in ISO 10993-12.

### Experimental Steps:

1) 1 cm² square samples of human fascia were dissected and placed in the wells of a low attachment 6 wells plate (total of 21 cm²/wells), 1 wells/donors (Costar, #3471).
2) The total weight of the samples were recorded and 7 mL of extraction media were added to each well (L-929 cell expansion media):
   - α-MEM (Gibco, #12571)
   - 10 % fetal bovine serum
   - 1 % penicillin/streptomycin
   *Note: Presence of fetal bovine serum should enable the extraction of polar and non-polar leachable materials. According to the ISO 10993-12, the extraction volume should be of 1mL for 3cm² of samples (thickness > 0.5 mm).*
2) Samples were then placed on a rocker and incubated for 24 hours at 37°C in an incubator (>90% humidity and 5% CO₂ atmosphere). Media alone was also incubated for L-929 controls and positive (SDS) controls.
3) 20,000 sub-confluent L-929 cells/wells were plated in a black, clear-bottom, 96 well plate (Tissue culture treated, Costar #3904) in a final volume of 100 µL of expansion media. Another black plate was seeded with a density of 10,000 cells/ well to ensure the use of subconfluent cells at the time of assay (i.e. day 3).
4) The extraction media was collected in 15 mL Falcon tubes and centrifuged at 300g for 10 minutes in order to remove big particles/cells extracted from the Fascia (Fascia being a frozen graft, cells were indeed observed in the extraction media if this step was not performed, resulting in a bias in the following Cyquant® assay).
5) The supernatant of the extraction media was applied to the L-929 cells plated at day 1 (removal of expansion media and addition of 100 µL of extraction media/well). At the same time, negative, positive and blank were performed:
   - Negative control: Cells in incubated expansion media (5 wells).
   - Positive control: Cells, 0.1 and 0.2 mg/ml of SDS (Sodium Dodecyl Sulfate) in incubated expansion media (5 wells/each concentration of SDS).
   - Experimental variable: Cells and extraction media (5 wells/donors).
   - Blank: Incubated expansion media only, no cells, no tissue (2 wells for every single condition).
6) Plates were incubated for another 24 hours at 37°C in an incubator.
   (Note: To make a calibration curve, L-929 cells were plated from concentrations ranging from 0 to 60,000 cells per well of a black, clear bottom, 96 wells plate.)
7) Qualitative assessments of cell morphology were performed by microscopic observations prior to running the healthy cells quantitation using the CyQUANT® Direct Cell Proliferation Assay (Invitrogen, #35012).
   *Note: all the following steps were performed avoiding a direct exposure to light.*
8) Cyquant® reagent was prepared according to the following recipe (For 12 ml of reagent):
   - 11.7 mL PBS
   - 48 µL CyQUANT® Direct nucleic acid stain
   - 240 µL CyQUANT® Direct background suppressor
9) The previous solution was well-mixed before adding 100 µL of it to every wells of the plate containing the L-929 cells.
10) The plate was then incubated for 1 hour at 37°C in an incubator.
11) Air bubbles (if any) were removed using a 26 gauge needle to avoid interferences.
12) Fluorescence was read at excitation/emission wavelength of 485/535 nm in a bottom reading mode (SpectraMax Gemini EM, Molecular Devices)

### Results:

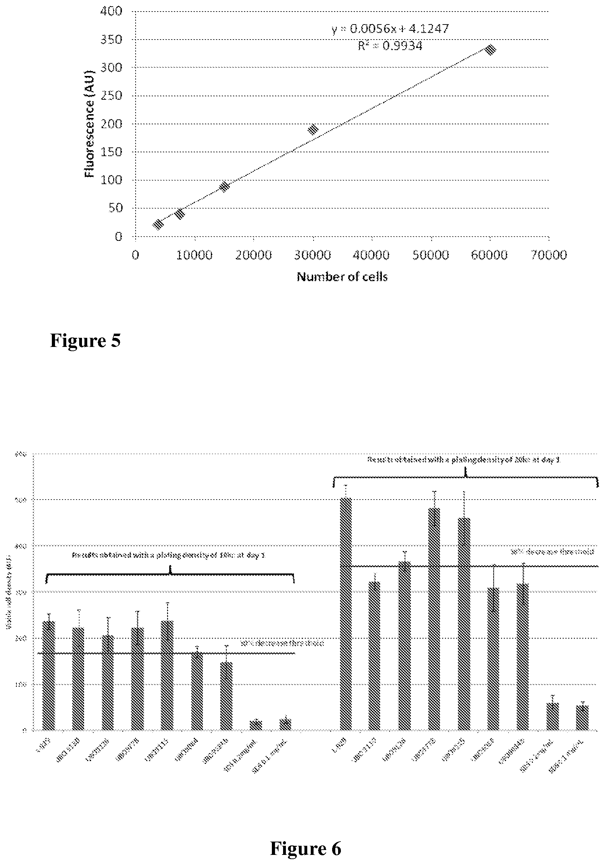

As can be observed in Figure 5, the fluorescent signal was proportional to the cell density, with a good linear response. The results obtained with the positive controls and the experimental groups tested are summarized in Figure 6.

As can be seen on Figure 6, exposure of the L-929 cells to the extraction media during 24 hours had a small effect on the healthy cell density. It is important to note that the cell density used greatly affected the results obtained. Indeed, cells seemed to be more sensitive to inhibitory compounds when plated at 20kc.

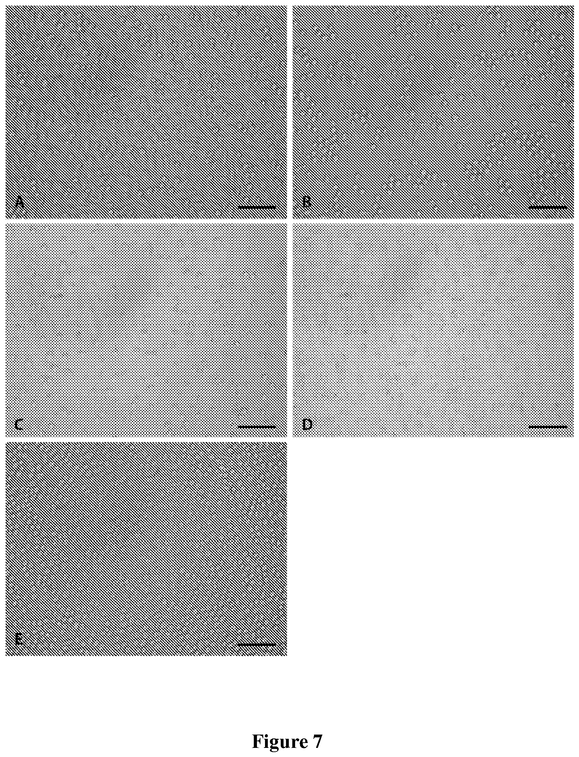

It is important to note that the quantification results obtained with the Cyquan®t test were hardly correlated to the microscopic observations in Figure 7: control cells (A, and higher magnification B)were dense and well-spread on the surface of the plastic wells. Cells exposed to extraction media (C, and higher magnification D) were less dense and a high fraction of them were rounded, despite the Cyquant® assay seems to indicate these cells still had a healthy membrane compared to cells exposed to SDS (0.1 mg/mL SDS (E) or 0.2 mg/mL (F)).

### Conclusion:

According to the criteria described in the ISO 10993-5, quantification tests resulting in more than 30% of decrease in cell viability is considered as a cytotoxic or cellular inhibition effect. The averaged results obtained in the 5 donors of this experiment were precisely on the borderline of this threshold of 30% (slightly less for one of the fascia processed from donor UBO9084).

The ISO documents also provide directions regarding a qualitative morphological assessment of cell toxicity/inhibition (see Table 4).

**Table 4 - Qualitative morphological grading of cytotoxicity of extracts**

| Grade | Reactivity | Conditions of all cultures |
|---|---|---|
| 0 | None | Discrete Intracytoplasmatic granules, no cell lysis, no reduction of cell growth |
| 1 | Slight | Not more than 20 % of the cells are round. loosely attached and without intracytoplasmatic granules, or show changes in morphology; occasional lysed cells are present; only slight growth Inhibition observable. |
| 2 | Mild | Not more than 50 % of the cells are round, devoid of intracytoplasmatic granules, no extensive cell lysis; not more than 50 % growth inhibition observable. |
| 3 | Moderate | Not more than 70 % of the cell layers contain rounded cells or are lysed: cell layers not completly destroyed, but more than 50 % growth inhibition observable. |
| 4 | Severe | Nearly complete or complete destruction of the cell layers. |

According to this table, despite a growth inhibition of approximately 30%, more than 50% of the cells were rounded after exposure to the extraction media, so that we can reasonably conclude that elements present in normal tissue can be inhibitory to cellular infiltration into such tissues and that removal of these inhibitory elements should facilitate a regenerative nature to processed tissues.

### Example 9: Biocompatibility of micronized dermal tissue matrix

The purpose of this experiment is to test and document the biocompatibility of micronized dermis produced by the process of the present invention. Freeze-dried micronized dermis samples were prepared according to the process as described herein to obtain micronized dermis. Biocompatibility was assessed by direct contact of the samples with L-929 cells (ATCC #CCL-1, source: *Mus musculus)* for two months. Qualitative assessment of cell morphology was performed by microscopic observation. A quantitative assessment of healthy cell density was performed using the CyQUANT® Cell Proliferation Assay.

### Procedure:

*Note: all procedures were carried out in a laminar flow hood in aseptic conditions. The following materials and conditions have been adapted from the recommendations described in ISO 10993-12 and ISO 10993-xx.*

### Day 1

Freeze-dried acellular, micronized dermis samples (Batch 2) were prepared according to the process of the present invention. Micronized dermis from donor BO39698, UBO 0099050202-12, particle size tested 25-300 µm.

The micronized dermis was weighed and then mixed with prepared media, α-MEM Gibco (lot #1045867), with 10% Fetal Bovine Serum, PAA (lot #A20411-7008), and 1% penicillin-streptomycin (Sigma, 051M0853), vortexed and placed on a 6 well, ultra low attachment plate (Corning, Costar #3471, lot #08711003, expiration date 03/27/2014), according to Table 5.

**Table 5**

| **Sample** | **Weight (g)** | **Media (ml)** |
|---|---|---|
| UMTB micronized dermis (3) | 0.003/each | 1/each |
| Controls,UMTB micronized dermis (2) | 0.003/each | 1/each |

L-929 sub-confluent, P5 culture was split and counted according to standard protocols.

Three of the micronized dermis samples were seeded with 800,000 L-929 cells, two samples were used as control with no cells. The samples were incubated for 2 months at 37°C, 5% CO₂, ≥90% humidity (equipment # 08-016). Media alone was also placed in the same plate and incubated under the same conditions.

### Days 2-55

Half the media was changed twice a week. Photographs were taken at days 10, 16 and 30.

### Day 57

L-929 cultures (P6) were harvested and split according to standard protocol and 300,000 cells in 2 ml of freshly prepared media were placed in a centrifuge tube. These cells were later used for a calibration curve. The cells, along with one seeded micronized dermis sample and one control were centrifuged. The supernatant was discarded and the pellets frozen at -80°C.

### Day 60

Two micronized dermis samples and one control were collected, centrifuged, the supernatant discarded and the pellets frozen at -80 °C.

### Day 61

A quantitative assessment of healthy cell density was performed using the CyQUANT® Cell Proliferation Assay kit (lot # 1050079), C7026.
1) CYQUANT® reagent was prepared by mixing 7.6 ml of H₂O, 400 µl buffer and 100 µl Cyquant. The lysis buffer obtained was dispensed in the following manner:
   - 200 µl was added to wells A1-H1 in a black 96 well plate (Corning Costar 3650, lot #24211045, exp.date 8/30/2013) for the calibration curve.
   - 200 µl was added to the cell pellets in a centrifuge tube (300,000 at -80°C) for the calibration curve
   - 800 µl was added to a sample of micronized dermis (control) in a black 96 well plate (3 wells)
   - 800 µl was added to the samples of micronized dermis seeded with L-929 cells in a black 96 well plate (6 wells, 3 each)
2) The samples were vortexed.
3) Pipet softly, homogenize and add 200 µl of the cell suspension into well A1.
4) Pipet 200 µl of the solution from A1 into B1 to create a calibration curve.
5) Pipet softly and homogenize without creating bubbles, continue the procedure to well G1 (H1=blank).
6) Pipet 200 µl of the solution in G1 and discard.
7) Vortex slightly and plate the samples and control.
8) Protect from the light, cover the plate in Aluminum foil.
9) Incubate for 5-15 minutes at room temperature.
10) Fluorescence was read at excitation/emission wavelength of 480/520 nm in a top reading mode using a Molecular Devices Flex Station 3.

### Data Analysis and Interpretation:

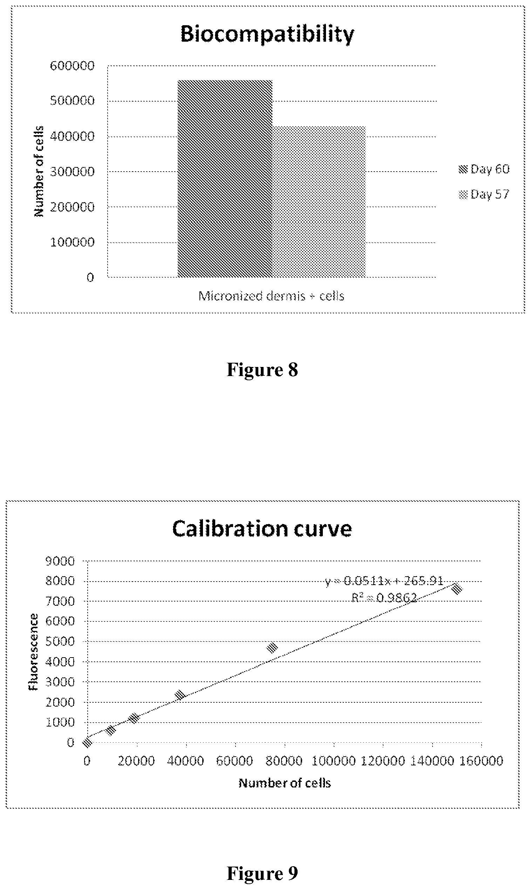

The samples collected in Day 60 show a larger number of cells then those collected in Day 57, as expected. The L-929 cells proliferated and survived during 60 days attached to the micronized dermis matrix showing that this matrix is biocompatible. See Table 6 below and Figures 8 and 9 for this quantitative data.

**Table 6**

| **Samples** | **Number of cells** | **Standard Deviation** | A cceptance Criteria | |
|---|---|---|---|---|
| | | | Pass | Fail |
| **Micronized dermis+ L-929 (Day 60)** | 558,875 | 7,198 | | |
| **Micronized dermis+ L-929 (Day 57)** | 429,054 | 23,107 | | |
| **L-929 Positive control (seeded Day 1)** | 800,000 | N/A | | |
| **Negative controls** | **Number of cells** | **Standard Deviation** | Acceptance Criteria | |
| | | | Pass 0 | Fail 0 |
| **Micronized dermis** | 0 | N/A | Yes | |

### Qualitative analysis:

The samples were examined microscopically and digital microphotographs were taken of every condition with the objective of illustrating cellular proliferation and adherence of the cells to the dermal matrix. All photomicrographs were taken at 100X unless otherwise noted. Similar microphotographs were taken of micronized dermis samples where no L-929 cells were seeded in order to serve as controls.

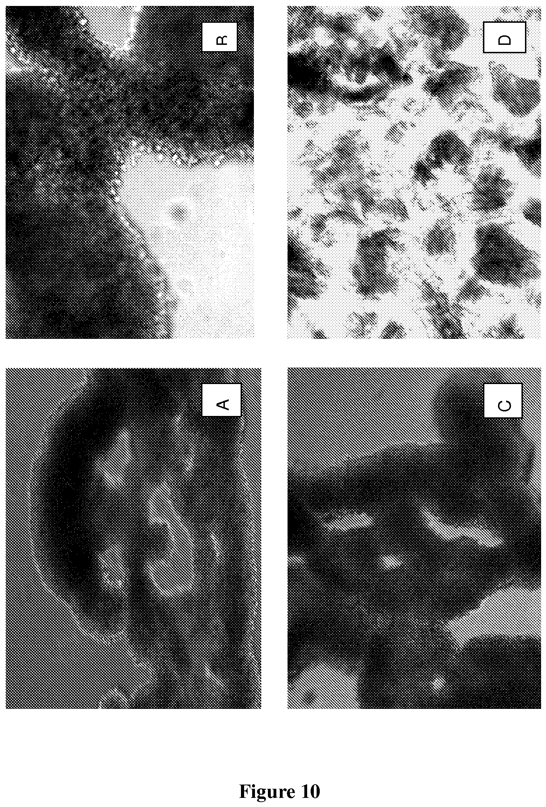

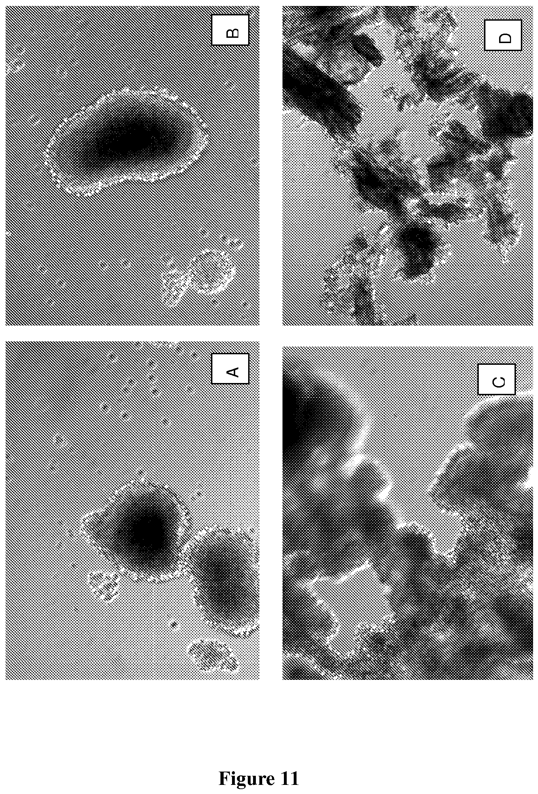

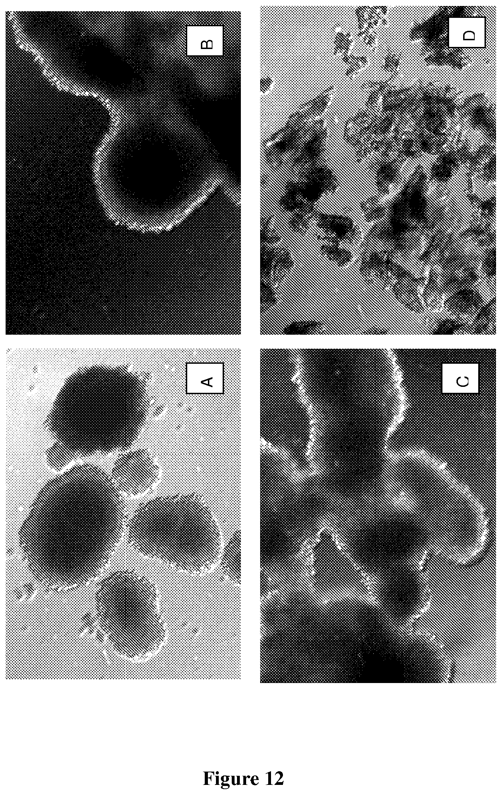

At Day 10, shown in Figure 10, the L-929 cells appear healthy appeared healthy and aggregated on the surface of the micronized dermis (A-C). Detail at 200X is also shown (B). Negative control (D) shows just micronized dermis 25-300 µm. This continues at Day 16, shown in Figure 11. By Day 30, shown in Figure 12, the cells remain healthy and aggregated to the surface of the micronized dermis (A-C).

### Conclusions:

The micronized dermis produced by the process of the present invention has a matrix structure that is biocompatible and provides an environment which is suitable and appropriate for cellular attachment and proliferation.

### Example 10: Resorption of micronized dermal tissue matrix upon injection into nude mice.

The purpose of this study was to examine the resorption over four weeks of the micronized dermal matrix product injected subcutaneously on either side of the spine of a nude mouse.

It was hypothesized that the dermal matrix product should not be quickly resorbed in a nude mouse, using a competitive product as a positive control. The size of the injection site should not change significantly. However, this measure is fairly subjective. Histologic evidence of resorption will be examined as well.

### Materials and Methods:

Each mouse was injected subcutaneously with test and control article. The injection sites were evaluated by palpation and measurement at termination. Body weights were obtained at injection, daily for the first week, then twice during each week thereafter, with weights collected at termination. Animals were observed cageside daily for signs of general clinical health.

At the end of the scheduled duration, the designated animals were euthanized. The injection sites were palpated and measured and the surrounding tissue was surgically excised. The tissues were placed in formalin for fixation. The samples were then stained with H&E, Alcian blue, trichrome and for elastin.

The mouse is suggested as an appropriate animal model for evaluating biocompatibility by the current ISO testing guidelines. The group size is based on the minimum amount of animals required for biological evaluation. The number of animals was chosen to provide some statistical significance in the results.

The subcutaneous injection route of exposure was selected because it is the intended route of administration to humans.
*Note: all procedures were carried out in an aseptic working environment. The following materials and conditions were adapted from the recommendations described in ISO 10993-12.*

### Results:

### Palpations and Measurements

**Table 6: Avg. area per measurement time point**

| **Group** | **Days Post-Implant** | | | | | | | |
|---|---|---|---|---|---|---|---|---|
| | 0 | 2 | 4 | 6 | 13 | 20 | 27 | 28 |
| Control | 140.1 | 154.9 | 170 | 166.3 | 168 | 201.7 | 174.5 | 171.2 |
| Test | 146.2 | 174 | 189.3 | 183.4 | 188.6 | 213.5 | 147.9 | 190.7 |

### Histologic Observations

**Table 7**

| | | **Chronic Inflammation** | **Foreign Material** | **Giant Cells** | **Elastin Disarray** | **Acute Inflammation** | **Overall Response** |
|---|---|---|---|---|---|---|---|
| **Animal** | **Side** | **0-4** | **0-4** | **0-4** | **0-4** | **0-4** | **0-4** |
| **1** | **Control** | 1 | 2 | 0 | 0 | 0 | 1 |
| **1** | **Test** | 1 | 1 | 0 | 0 | 0 | 1 |
| **2** | **Control** | 2 | 1 | 0 | 0 | 0 | 2 |
| **2** | **Test** | 1 | 1 | 0 | 0 | 0 | 2 |
| **3** | **Control** | 1 | 1 | 0 | 0 | 0 | 1 |
| **3** | **Test** | 1 | 0 | 0 | 0 | 0 | 1 |

### General Observations:

The control article implant was myxoid with sparse cellularity. In the test article implant, collagen is more dense and less cellular.

### Conclusions:

Both the control article and the test article show the evolution of a subdermal pseudocapsule with mild inflammation. Neither shows a significant inflammatory or giant cell response. The control article consists of loose, vaguely myxoid, fibroconnective tissue. Angiogenesis is more developed in the control article than on the test article. The test article consists of disorganized, more mature collagen with quiescent fibroblasts. Accordingly, the test article is more "active".

### Example 11: Wound healing clinical data using dermal tissue matrix as graft

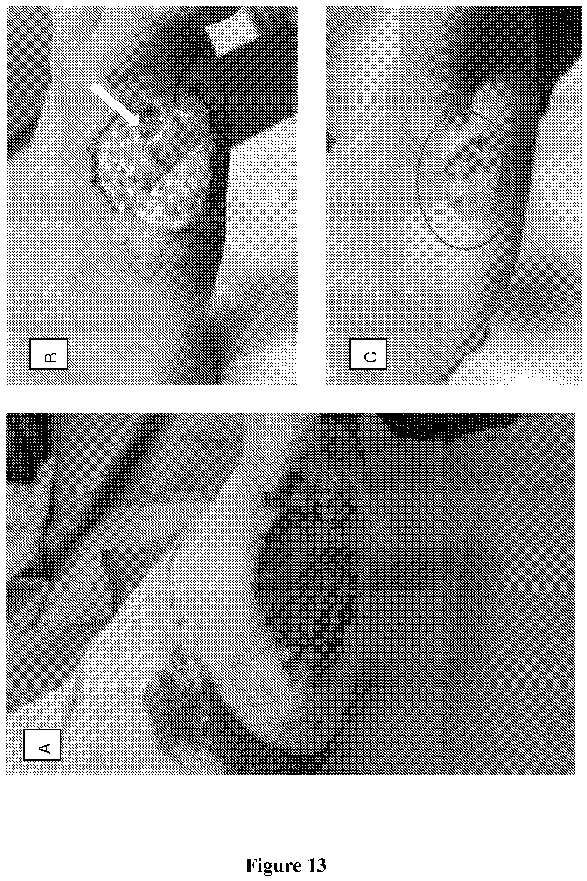

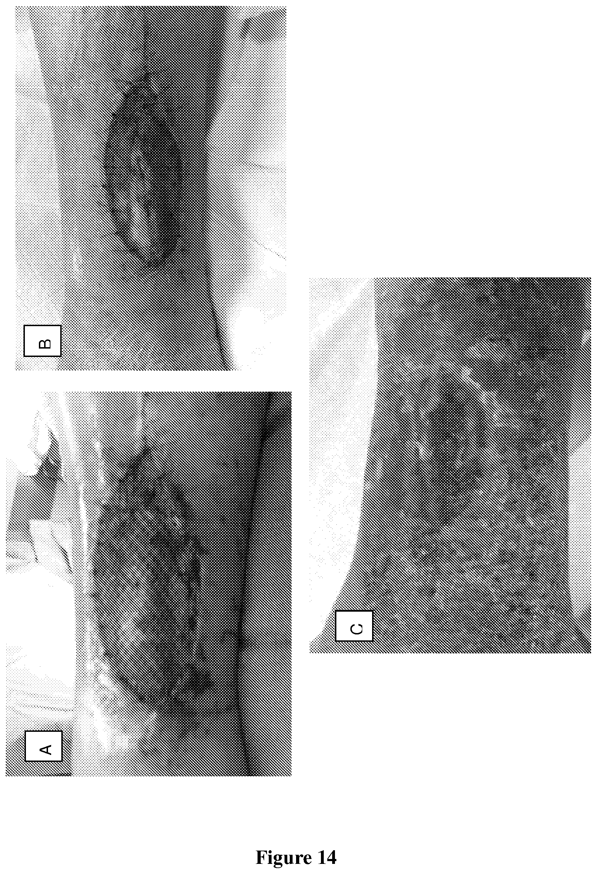

Dermal tissue matrices in the form of tissue grafts were supplied to various clinicians to test whether the grafts could promote regenerative repair of soft tissue in *in situ* applications to humans. The clinicians followed their own procedures and protocols for applying and/or using the dermal matrix tissue grafts on wounds of their patients. At least two provided data of their results, shown in Figures 13 and 14.

Specifically, in Figure 13 a 4x4cm graft of dermal matrix, such as a dermal sheet, was placed on a wounded area of skin (panel A). By Day 12 (panel B), the wound was already beginning to heal, as evident from granulation tissue developing at the site, as well as from reduced wound size. By Day 40 (panel C), the wound was almost fully healed. More than simply healing, regeneration was occurring, as indicated by the lack of scarring in the tissue.

Figure 14, taken from a different patient, also shows regenerative repair. At Day 1 (panel A), a graft of dermal matrix was placed on a post-radiation wound. By the following day (panel B), cellular infiltration was already observable. By Day 30 (panel C), regeneration of the wounded tissue was well underway.

## Claims

1. A process for preparing a tissue matrix suitable for regenerative repair of soft tissue, comprising:
contacting an isolated portion of connective tissue with a surfactant,
contacting the connective tissue with a disinfectant, reducing at least one of proteoglycans, lipids, phospholipids, nucleic acids, and major histocompatibility (MHC) antigens that inhibit cellular infiltration and attachment, and dehydrating the resultant tissue matrix for storage, and
further comprising reducing the proteoglycan content of the connective tissue by a range of 30% to 50%, such that the tissue matrix retains 50% to 70% of extractable proteoglycans.

2. The process as recited in claim 1 further comprising reducing the lipid and phospholipid content of the connective tissue by at least 70%, preferably by a range of 70% to 95%.

3. The process as recited in claim 1 further comprising reducing the nucleic acid content of the connective tissue by at least 30%, preferably by a range of 30% to 90%.

4. The process as recited in claim 1 further comprising reducing the major histocompatibility (MHC) antigen I and II content of the connective tissue by at least 85%, preferably by a range of 85% to 99%.

5. The process as recited in claim 1 further comprising removing endotoxins from the connective tissue.

6. The process as recited in claim 1 further comprising contacting the connective tissue with a chondroitinase enzyme, preferably chondroitinase ABC, to reduce at least one of proteoglycans, lipids, phospholipids, major histocompatibility antigens (MHC) I and II, and endotoxins in the connective tissue.

7. The process as recited in claim 1 further comprising contacting the connective tissue with an endonuclease to reduce nucleic acids in the connective tissue and with a lipase enzyme to reduce at least one of lipids, phospholipids, and endotoxins in the connective tissue.

8. The process as recited in claim 1 wherein said surfactant comprises at least one of an anionic surfactant, a nonionic surfactant, a cationic surfactant, and combinations thereof, wherein preferably said surfactant is a detergent comprising at least one of an anionic detergent, a nonionic detergent, a cationic detergent, and combinations thereof.

9. The process as recited in claim 1 wherein said disinfectant comprises at least one of peracetic acid, chlorine dioxide, hydrogen peroxide, polyvinylpyrolidineiodide , formaldehyde, glutaraldehyde , phenoxyethanol , methylparaben , propylparaben, sodium hydroxymethylglycinate, diazolidinyl urea, DMDM hydantoin, iodopropynyl butylcarbamage, propylene glycol, and combinations thereof.

10. A tissue matrix obtainable by the process of anyone of claims 1-9,
for use in regenerative repair of soft tissue, said tissue matrix comprising: a scaffold portion structured to provide shape to the tissue matrix, and a non-structural portion disposed in interspersed relation through at least a part of said scaffold portion, wherein said scaffold portion and said non-structural portion are collectively structured to promote cellular infiltration, attachment, and proliferation of at least one host cell into the tissue matrix upon implantation or transplantation of the tissue matrix into a host tissue, wherein said scaffold portion and said non-structural portion comprise reduced levels of at least one of proteoglycans, lipids, phospholipids, nucleic acids, major histocompatibility (MHC) antigens, and endotoxins, wherein the proteoglycan levels are reduced by a range of 30% to 50%, such that the tissue matrix retains 50% to 70% of extractable proteoglycans.

11. The tissue matrix as recited in claim 10 wherein at least one of said structural portion and said non-structural portion have been at least partially acellularized.

12. A kit for regenerative soft tissue repair, said kit comprising : a tissue matrix of isolated connective tissue according to anyone of claims 10-11, said tissue matrix comprising reduced levels of at least one of proteoglycans, lipids, phospholipids, nucleic acids, major histocompatibility (MHC) antigens, and endotoxins, wherein said tissue matrix is structured to promote cellular infiltration, attachment, and proliferation of at least one host cell into the tissue matrix upon implantation or transplantation of the tissue matrix into a host tissue.

13. The kit as recited in claim 12 further comprising an amount of transfer solution for transferring said tissue matrix into a host tissue.

14. The kit as recited in claim 12 further comprising at least one assay reagent for use in detecting regenerative repair in a tissue, and said assay reagent being structured for use in detecting at least one of cellular infiltration, cellular attachment, cellular proliferation, cellular differentiation, and synthesized matrix materials in a tissue, and preferably being selected from at least one of a fluorescent compound, a molecular label, a molecular label substrate, an enzyme, a radioisotope, heavy atoms, a reporter gene, a vector, a luminescent compound, and an antibody.

## Patentansprüche

1. Verfahren zur Herstellung einer Gewebematrix, die zur regenerativen Reparatur von Weichgewebe geeignet ist, umfassend:
in Kontakt bringen eines isolierten Abschnitts eines Bindegewebes mit einem Tensid,
in Kontakt bringen des Bindegewebes mit einem Desinfektionsmittel, Reduzieren von mindestens einem von Proteoglykanen, Lipiden, Phospholipiden, Nukleinsäuren und Haupthistokompatibilitäts (MHC)-Antigenen, die zelluläre Infiltration und Anheftung inhibieren, und Dehydrieren der erhaltenen Gewebematrix für die Lagerung und
weiterhin umfassend Reduzieren des Proteoglykangehalts des Bindegewebes um einen Bereich von 30 % bis 50 %, so dass die Gewebematrix 50 % bis 70 % extrahierbare Proteoglykane enthält.

2. Verfahren nach Anspruch 1, weiterhin umfassend Reduzieren des Lipid- und Phospholipidgehalts des Bindegewebes um mindestens 70 %, vorzugsweise um einen Bereich von 70 % bis 95 %.

3. Verfahren nach Anspruch 1, weiterhin umfassend Reduzieren des Nukleinsäuregehalts des Bindegewebes um mindestens 30 %, vorzugsweise um einen Bereich von 30 % bis 90 %.

4. Verfahren nach Anspruch 1, weiterhin umfassend Reduzieren des Haupthistokompatibilitäts (MHC)-Antigen-I- und -II-Gehalts des Bindegewebes um mindestens 85%, vorzugsweise um einen Bereich von 85 % bis 99 %.

5. Verfahren nach Anspruch 1, ferner umfassend das Entfernen von Endotoxinen aus dem Bindegewebe.

6. Verfahren nach Anspruch 1, weiterhin umfassend in Kontakt bringen des Bindegewebes mit einem Chondroitinaseenzym, vorzugsweise mit Chondroitinase-ABC, um mindestens eines von Proteoglykanen, Lipiden, Phospholipiden, Haupthistokompatibilitäts (MHC) Antigenen I und II und Endotoxinen im Bindegewebe zu reduzieren.

7. Verfahren nach Anspruch 1, weiterhin umfassend in Kontakt bringen des Bindegewebes mit einer Endonuklease, um Nukleinsäuren im Bindegewebe zu reduzieren, und mit einem Lipaseenyzm, um mindestens eines von Lipiden, Phospholipiden und Endotoxinen in dem Bindegewebe zu reduzieren.

8. Verfahren nach Anspruch 1, wobei das Tensid mindestens eines von einem anionischen Tensid, einem nichtionischen Tensid, einem kationischen Tensid und Kombinationen davon umfasst, wobei vorzugsweise das Tensid ein Detergens ist, umfassend mindestens eines von einem anionischen Detergens, einem nichtionischen Detergens, einem kationischen Detergens und Kombinationen davon.

9. Verfahren nach Anspruch 1, wobei das Desinfektionsmittel mindestens eines von Peressigsäure, Chlordioxid, Wasserstoffperoxid, Polyvinylpyrrolidiniodid, Formaldehyd, Glutaraldehyd, Phenoxyethanol, Methylparaben, Propylparaben, Natriumhydroxymethylglycinat, Diazolidinylharnstoff, DMDM-Hydantoin, lodpropinylbutylcarbamat, Propylenglycol und Kombinationen davon umfasst.

10. Gewebematrix, erhältlich durch das Verfahren nach einem der Ansprüche 1 bis 9,
zur Verwendung bei der regenerativen Reparatur von Weichgewebe, wobei die Gewebematrix umfasst: einen Gerüstabschnitt, der strukturiert ist, um der Gewebematrize Form zu verleihen, und einen nichtstrukturierten Abschnitt, der durch mindestens einen Teil des Gerüstabschnitts verteilt angeordnet ist, wobei der Gerüstabschnitt und der nichtstrukturierte Abschnitt kollektiv strukturiert sind, um die zelluläre Infiltration, Anheftung und Proliferation von mindestens einer Wirtszelle in die Gewebematrix nach Implantation oder Transplantation der Gewebematrix in ein Wirtsgewebe zu fördern, wobei der Gerüstabschnitt und der nichtstrukturierte Abschnitt reduzierte Mengen von mindestens einem von Proteoglykanen, Lipiden, Phospholipiden, Nukleinsäuren, Haupthistokompatibilitäts (MHC)-Antigenen und Endotoxinen umfassen, wobei die Proteoglykangehalte um einen Bereich von 30 % bis 50 % reduziert sind, so dass die Gewebematrix 50 % bis 70 % extrahierbare Proteoglykane enthält.

11. Gewebematrix nach Anspruch 10, wobei mindestens einer von dem strukturierten Abschnitt und dem nichtstrukturierten Abschnitt zumindest teilweise azellularisiert sind.

12. Kit für regenerative Weichgewebereparatur, wobei das Kit umfasst: eine Gewebematrix aus isoliertem Bindegewebe gemäß einem der Ansprüche 10 oder 11, wobei die Gewebematrix reduzierte Mengen von mindestens einem von Proteoglykanen, Lipiden, Phospholipiden, Nukleinsäuren, Haupthistokompatibilitäts (MHC)-Antigenen und Endotoxinen umfasst, wobei die Gewebematrix so strukturiert ist, dass sie die zelluläre Infiltration, Anheftung und Proliferation von mindestens einer Wirtszelle in die Gewebematrix bei der Implantation oder Transplantation der Gewebematrix in ein Wirtsgewebe fördert.

13. Kit nach Anspruch 12, weiterhin umfassend eine Menge an Übertragungslösung zum Überführen der Gewebematrix in ein Wirtsgewebe.

14. Kit nach Anspruch 12, weiterhin umfassend mindestens ein Assay-Reagens zur Verwendung bei der Detektion einer regenerativen Reparatur in einem Gewebe, wobei das Assay-Reagens zur Verwendung beim Nachweis von mindestens einem von zellulärer Infiltration, zellulärer Bindung, zellulärer Proliferation, zellulärer Differenzierung und synthetisierten Matrixmaterialien in einem Gewebe strukturiert ist, und vorzugsweise aus einer fluoreszierenden Verbindung, einer molekularen Markierung, einem molekularen Markersubstrat, einem Enzym, einem Radioisotop, schweren Atomen, einem Reportergen, einem Vektor, einem Lumineszenzmittel und einem Antikörper ausgewählt ist.

## Revendications

1. Procédé de préparation d'une matrice tissulaire convenant à une réparation régénérative d'un tissu mou, comprenant :
la mise en contact d'une partie isolée de tissu conjonctif avec un tensioactif,
la mise en contact du tissu conjonctif avec un désinfectant, la réduction d'au moins l'un de protéoglycanes, de lipides, de phospholipides, d'acides nucléiques et d'antigènes d'histocompatibilité majeure (MHC) qui empêchent une infiltration et une fixation cellulaires et la déshydratation de la matrice tissulaire obtenue pour un stockage, et
comprenant en outre la réduction de la teneur en protéoglycanes du tissu conjonctif d'une plage de 30 % à 50 % de sorte que la matrice tissulaire retienne 50 % à 70 % de protéoglycanes extractibles.

2. Procédé selon la revendication 1, comprenant en outre la réduction de la teneur en lipides et en phospholipides du tissu conjonctif d'au moins 70 %, de préférence d'une plage de 70 % à 95 %.

3. Procédé selon la revendication 1, comprenant en outre la réduction de la teneur en acides nucléiques du tissu conjonctif d'au moins 30 %, de préférence d'une plage de 30 % à 90 %.

4. Procédé selon la revendication 1, comprenant en outre la réduction de la teneur en antigènes d'histocompatibilité majeure (MHC) I et Il du tissu conjonctif d'au moins 85 %, de préférence d'une plage de 85 % à 99%.

5. Procédé selon la revendication 1, comprenant en outre l'élimination d'endotoxines du tissu conjonctif.

6. Procédé selon la revendication 1, comprenant en outre la mise en contact du tissu conjonctif avec un enzyme chondroïtinase, de préférence chondroïtinase ABC, pour réduire au moins l'un(e) de protéoglycanes, de lipides, de phospholipides, d'antigènes d'histocompatibilité majeure (MHC) I et II et d'endotoxines dans le tissu conjonctif.

7. Procédé selon la revendication 1, comprenant en outre la mise en contact du tissu conjonctif avec une endonucléase pour réduire les acides nucléiques dans le tissu conjonctif et avec une enzyme lipase pour réduire au moins l'un(e) de lipides, de phospholipides et d'endotoxines dans le tissu conjonctif.

8. Procédé selon la revendication 1, dans lequel ledit tensioactif comprend au moins l'un d'un tensioactif anionique, d'un tensioactif non ionique, d'un tensioactif cationique et de combinaisons de ceux-ci, dans lequel, de préférence, ledit tensioactif est un détergent comprenant au moins l'un d'un détergent anionique, d'un détergent non ionique, d'un détergent cationique et de combinaisons de ceux-ci.

9. Procédé selon la revendication 1, dans lequel ledit désinfectant comprend au moins l'un parmi l'acide peracétique, le dioxyde de chlore, le peroxyde d'hydrogène, le poly(iodure de vinylpyrolidine), le formaldéhyde, le glutaraldéhyde, le phénoxyéthanol, le méthylparaben, le propylparaben, l'hydroxyméthylglycinate de sodium, la diazolidinylurée, l'hydantoïne DMDM, le butylcarbamate d'iodopropynyle, le propylèneglycol, et des combinaisons de ceux-ci.

10. Matrice tissulaire qui peut être obtenue par le procédé de l'une quelconque des revendications 1 à 9, pour utilisation dans la réparation régénérative d'un tissu mou, ladite matrice tissulaire comprenant : une portion de support structurée pour donner une forme à la matrice tissulaire, et une portion non structurelle disposée en relation intercalée à travers au moins une partie de ladite portion de support, dans laquelle ladite portion de support et ladite portion non structurelle sont collectivement structurées pour promouvoir une infiltration, une fixation et une prolifération cellulaires d'au moins une cellule hôte dans la matrice tissulaire lors de l'implantation ou de la transplantation de la matrice tissulaire dans un tissu hôte, dans laquelle ladite portion de support et ladite portion non structurelle comprennent des niveaux réduits d'au moins l'un(e) de protéoglycanes, de lipides, de phospholipides, d'acides nucléiques, d'antigènes d'histocompatibilité majeure (MHC) et d'endotoxines, dans laquelle les niveaux de protéoglycanes sont réduits d'une plage de 30 % à 50 % de sorte que la matrice tissulaire retienne 50 % à 70 % de protéoglycanes extractibles.

11. Matrice tissulaire selon la revendication 10, dans laquelle au moins l'une de ladite portion structurelle et de ladite portion non structurelle ont été au moins en partie acellularisées.

12. Kit pour la réparation régénérative de tissus mous, ledit kit comprenant une matrice tissulaire de tissu conjonctif isolé selon l'une quelconque des revendications 10 à 11, ladite matrice tissulaire comprenant des niveaux réduits d'au moins l'un(e) de protéoglycanes, de lipides, de phospholipides, d'acides nucléiques, d'antigènes d'histocompatibilité majeure (MHC) et d'endotoxines, dans lequel ladite matrice tissulaire est structurée pour promouvoir une infiltration, une fixation et une prolifération cellulaires d'au moins une cellule hôte dans la matrice tissulaire lors de l'implantation ou de la transplantation de la matrice tissulaire dans un tissu hôte.

13. Kit selon la revendication 12, comprenant en outre une quantité de solution de transfert pour transférer ladite matrice tissulaire dans un tissu hôte.

14. Kit selon la revendication 12, comprenant en outre au moins un réactif de dosage pour utilisation dans la détection d'une réparation régénérative dans un tissu, et ledit réactif de dosage étant structuré pour une utilisation dans la détection d'au moins l'une d'une infiltration cellulaire, d'une fixation cellulaire, d'une prolifération cellulaire, d'une différentiation cellulaire et de matériaux matriciels synthétisés dans un tissu, et étant de préférence choisi parmi au moins l'un(e) d'un composé fluorescent, d'une étiquette moléculaire, d'un substrat d'étiquette moléculaire, d'une enzyme, d'un radioisotope, d'atomes lourds, d'un gène rapporteur, d'un vecteur, d'un composé luminescent et d'un anticorps.
